# FASCICULE DE BREVET EUROPEEN

(11) **EP 0 702 723 B1**
(45) Date de publication et mention de la délivrance du brevet: **04.09.2002**
(21) Numéro de dépôt: 94914431.5
(22) Date de dépôt: 21.04.1994
(51) Int. Cl.: C12N 15/86, A61K 48/00, C12N 5/10, A61L 27/00, A61K 9/14, C12N 9/24, C12N 15/27, C12N 9/12, C12N 15/12, C12N 15/56, C12N 15/54

(54) **IMPLANT BIOCOMPATIBLE POUR L'EXPRESSION ET LA SECRETION IN VIVO D'UN COMPOSE THERAPEUTIQUE**
BIOKOMPATIBLES IMPLANTAT ZUR IN VIVO EXPRESSION UND SEKRETION VON THERAPEUTISCHER VERBINDUNG
BIOCOMPATIBLE IMPLANT FOR THE EXPRESSION AND SECRETION IN VIVO OF A THERAPEUTICAL COMPOUND

(30) Priorité: 21.04.1993 FR 9304700; 26.07.1993 FR 9309185
(43) Date de publication de la demande: 27.03.1996
(60) Demande divisionnaire: 02003797.4
(73) Titulaire: INSTITUT PASTEUR, 75724 Paris Cédex 15 (FR)
(72) Inventeur: MOULLIER, Philippe, F-92100 Meudon (FR); DANOS, Olivier, F-92380 Garches (FR); HEARD, Jean-Michel, F-75014 Paris (FR); FERRY, Nicolas, F-75013 Paris (FR)
(74) Mandataire: Gutmann, Ernest
(86) Numéro de dépôt international: FR9400456
(87) Numéro de publication internationale: WO94024298

(56) Documents cités:
- EP-A- 0 178 220
- WO-A-90/02797
- WO-A-90/12603
- WO-A-91/10728
- WO-A-92/07943
- WO-A-92/15676
- WO-A-93/07913
- WO-A-93/08850
- FR-A- 2 460 657
- US-A- 4 980 289
- JOURNAL OF CELLULAR BIOCHEMISTRY vol. SUP0, no. 18A , 15 Janvier 1994 page 240 SALVETTI, A. ET AL 'Retroviral vectors for the in vivo delivery of alpha-l-iduronidase and aryl-sulfatase b in human mucopolysaccharidoses type I and VI' & Keystone symposium on gene therapy Copper mountain , USA 15 au 22 Janvier 1994
- JOURNAL OF CELLULAR BIOCHEMISTRY vol. SUP0, no. 18A , 15 Janvier 1994 page 239 MOULLIER, P. ET AL 'Implants of genetically modified skin fibroblasts for lysosomal enzyme delivery: studies in newborn mutant mice and in normal dogs' & Keystone symposium on gene therapy Copper mountain , USA 15 au 22 Janvier 1994
- JOURNAL OF CELLULAR BIOCHEMISTRY vol. SUPPL, no. 17E , 29 Mars 1993 page 225 DANOS, O. ET AL. 'Correction of lysosomal storage in beta-glucuronidase-deficient mice by genetically-modified cellular implants' & Keystone Symposium on gene therapy Keystone, Colorado, USA 12 au 18 Avril 1993
- MEDECINE-SCIENCES vol. 9, no. 2 , Février 1993 pages 208 - 210 DANOS, O. ET AL. 'Réimplantation de cellules génétiquement modifiées dans des néo-organes vascularisés'
- TRANSPLANTATION vol. 56, no. 2 , Août 1993 pages 427 - 432 MOULLIER, P. ET AL. 'Continuous systemic secretion of a lysosomal enzyme by genetically modified mouse skin fibroblasts'
- JOURNAL OF CELLULAR BIOCHEMISTRY vol. SUPPL, no. 15F , 1 Avril 1991 page 242 MOULLIER, P. ET AL. 'Organoid neovascular structure: effects of various matrix and angiogenic factors' & Meeting on FGF, endothelial cell growth factors and angiogenesis// 20th Annual Meeting of the Keystone Symposia on molecular and cellular biology, Keystone, USA, 1er au 7 Avril 1991
- TRANSPLANTATION vol. 56, no. 2 , Août 1993 pages 423 - 427 GILBERT, J.C. ET AL. 'Cell transplantation of genetically altered cells on biodegradable polymer scaffolds in syngeneic rats'
- PROCEEDINGS OF THE NATIONAL ACADEMY OF SCIENCES OF USA. vol. 85, no. 9 , Mai 1988 , WASHINGTON US pages 3150 - 3154 ST. LOUIS, D. & VERMA, I.M. 'An alternative approach to somatic cell gene therapy'
- JOURNAL OF CELLULAR BIOCHEMISTRY vol. SUPPL, no. 16F , 3 Avril 1992 page 62 MOULLIER, P. ET AL. 'Murine skin fibroblasts and bone marrow cells as targets for in vivo transfer and expression of the human beta-glucuronidase cDNA' & Keystone Symposium on gene transfer, replacement and augmentation Copper Mountain, Colorado, USA 3 au 9 Avril 1992
- SCIENCE vol. 241, no. 4871 , 9 Septembre 1988 , LANCASTER, PA US pages 1349 - 1352 THOMPSON, J.A. ET AL. 'Site-directed neovessel formation in vivo'

## Description

L'invention concerne un implant biocompatible pour l'expression chez l'homme ou chez l'animal de substances déterminées, notamment pour l'ancrage des cellules recombinantes de l'invention.

Des vecteurs rétroviraux pour la préparation de cellules recombinantes susceptibles d'être implantées in vivo dans un but thérapeutique sont également décrits.

### Etat de la technique

L'introduction in vivo d'implants susceptibles d'exprimer des substances déterminées, par exemple à des fins thérapeutiques, nécessite l'utilisation de moyens efficaces s'agissant de la finalité thérapeutique ou prophylactique recherchée et la capacité pour l'organisme chez lequel est introduit l'implant, de le tolérer à plus ou moins long terme.

La demande de brevet internationale antérieure publiée sous le numéro 92/15676, décrit des moyens pour réaliser in vivo l'expression de séquences de nucléotides déterminées, en vue d'un traitement thérapeutique de maladies résultant d'une anomalie génétique. Cette demande internationale 92/15676, propose d'utiliser des fibroblastes génétiquement modifiés par un vecteur rétroviral, en vue de les implanter dans le tissu conjonctif de la peau du sujet à traiter.

La séquence de nucléotides dont l'expression est recherchée dans cette demande internationale antérieure, est placée sous le contrôle de la séquence LTR (Long Terminal Repeat) du vecteur rétroviral et/ou sous le contrôle de promoteurs exogènes constitutifs ou inductibles, la séquence LTR du rétrovirus étant néanmoins conservée lorsque le promoteur exogène est présent.

La publication Danos et al, Médecine-Science 9 (2), Février 1993, pages 208-210, décrit des néoorganes susceptibles de contenir des cellules génétiquement modifiées, en vue de la réimplantation *in situ.*

Ce document ne suggère pas l'utilisation d'un support biocompatible à base de calcaire pour la préparation d'un implant.

La publication PNAS, vol. 85, pages 3150-3154, 1988, décrit l'utilisation de collagène pour enrober des cellules telles que des fibroblastes exprimant le facteur 9, dans le but de réaliser un implant utilisé chez la souris. Ce document ne suggère pas l'utilisation d'un matériau biocompatible pour la réalisation d'un implant.

La demande de brevet WO 93/08850 décrit un procédé de préparation d'une matrice artificielle pour l'implantation contrôlée de cellules, à partir de supports synthétiques.

### Résumé de l'invention

L'invention propose des moyens permettant de réaliser in vivo, l'expression d'une séquence de nucléotides choisie, dans des conditions telles que cette expression est obtenue sur une durée de plusieurs mois, de préférence plus de 6 mois, de telle façon que le produit de la séquence de nucléotides exprimée est présent en quantité suffisante et dans des conditions appropriées pour produire un effet thérapeutique recherché in vivo.

Les moyens définis dans la présente demande permettent de réaliser l'expression et la sécrétion d'une protéine, glycoprotéine, ou d'un peptide ayant un intérêt biologique, pour autoriser son utilisation thérapeutique in vivo.

L'invention concerne également l'utilisation de matériaux biocompatibles, en tant que supports pour l'introduction chez l'homme ou chez l'animal, de cellules, par exemple de cellules recombinantes de l'invention définies ci-dessous, à partir desquelles on souhaite produire une molécule déterminée, notamment à des fins thérapeutiques.

La préparation d'implants fait appel à de tels supports, propres à être mis au contact de cellules et de différents facteurs favorisant l'adhérence de ces cellules au support si nécessaire, dans des conditions telles que les différents constituants présents conservent leurs propriétés fonctionnelles et structurales naturelles principales.

Ces supports biocompatibles, d'origine biologique ou non peuvent être résorbables ou non résorbables par l'hôte chez lequel ils sont introduits.

Des vecteurs rétroviraux ayant la capacité d'infecter des cellules et en particulier des cellules eucaryotes ainsi que les cellules recombinantes comprenant ces vecteurs sont également décrits. Les cellules recombinantes peuvent être administrées, ou implantées chez un patient, et produisant ainsi in vivo une protéine ou tout produit d'expression d'une séquence de nucléotides déterminée, insérée dans vecteur rétroviral.

Les inventeurs ont examiné quels étaient les paramètres qui, à différents niveaux, permettraient d'exprimer in vivo une séquence de nucléotides déterminée, de façon à améliorer l'expression de la séquence de nucléotides insérée dans le vecteur afin d'obtenir un effet thérapeutique, si nécessaire de longue durée.

Ainsi, ils ont mis en évidence que lorsque la séquence de nucléotides exogène dont on cherche l'expression est avantageusement placée dans le vecteur destiné à l'infection des cellules, sous le contrôle d'un promoteur exogène constitutif ou inductible, la séquence LTR interne au vecteur rétroviral doit alors être partiellement supprimée. La délétion doit être suffisante pour affecter la transcription de l'ARNm.

### Description détaillée de l'invention

### Implant biocompatible

L'implant ou néo-organe de l'invention est obtenu par le procédé qui consiste en l'assemblage in vitro de différents éléments afin de former un implant qui est introduit de préférence dans la cavité péritonéale du receveur. D'autres sites d'implantation sont utilisables tels que l'espace péri-rénal, le derme. In vivo, l'implant donne naissance à un tissu conjonctif néo-formé, vascularisé, non modifié au cours du temps.

De façon générale, l'implant de l'invention est caractérisé en ce qu'il comprend :
- un support biocompatible à base de calcaire permettant l'ancrage biologique de cellules ;
- des cellules ayant la capacité d'exprimer et de secréter naturellement ou après recombination un composé déterminé, par exemple un composé ayant un intérêt thérapeutique ; et
- un constituant capable d'induire et/ou de favoriser la gélification desdites cellules.

Particulièrement, les éléments participant à l'assemblage de l'implant in vitro sont les suivants:
1) **Un support rigide.** Il peut être préparé à partir de biomatériau tel que le corail,
2) **Un gel de collagène.** On peut utiliser du collagène de queue de rat, du collagène bovin, du collagène humain ; et
3) **des cellules génétiquement modifiées,** de préférence les cellules recombinantes de la présente invention, qui sont décrites de façon détaillée plus loin.

L'invention a donc pour objet un implant ou néo-organe caractérisé en ce qu'il comprend un support rigide biocompatible, particulièrement un support rigide d'origine biologique, permettant l'ancrage biologique de cellules mises en contact, préalablement ou non avec des constituants capables d'induire et/ou de favoriser leur inclusion au sein d'une matrice formant un gel, lesdites cellules étant choisies pour leur capacité à exprimer et à sécréter naturellement ou après recombinaison un composé déterminé, par exemple un composé ayant un intérêt thérapeutique.

L'expression "ancrage biologique" signifie que les cellules contenues dans l'implant peuvent se fixer à la surface du support biocompatible ou, dans certains cas, pénétrer à l'intérieur de ce support.

Cette fixation des cellules sur le support est permise notamment par la présence des constituants capables d'induire et/ou de favoriser l'inclusion des cellules au sein d'une matrice ayant la constitution d'un gel.

Cette inclusion dans la matrice, appelée gélification permet l'organisation des cellules en une structure à trois dimensions dans un environnement amorphe, ne donnant pas lieu in vivo à une inflammation prolongée.

L'implant obtenu est selon un premier mode de réalisation de l'invention constitué d'une part d'un support biocompatible à base calcaire, en particulier à base de carbonate de calcium et, de préférence de corail et d'autre part d'un gel éventuellement chargé de cellules exprimant la substance d'intérêt, notamment de cellules recombinantes.

L'invention concerne également une méthode de préparation d'un implant. La méthode comprend les étapes de :
- mise en contact du support biocompatible à base de calcaire avec lesdites cellules et un constituant capable d'induire et/ou de favoriser leur gélification ;
- incubation de la préparation obtenue à l'étape précédente afin d'obtenir la gélification desdits constituants ;
- culture des cellules ainsi obtenues dans des conditions leur permettant de s'attacher aux constituants gélifiés, et
- récupération de l'implant ainsi obtenu.

### Implant comprenant un matériau biocompatible d'origine biologique.

De préférence, le matériau utilisé est un corail à haute porosité, c'est-à-dire qu'il possède une porosité telle que décrite dans le brevet français n° 2 460 657. Il peut s'agir aussi d'une poudre de porosité suffisante pour permettre d'obtenir une armature solide en vue de la constitution d'un réseau cellulaire comportant ou non un support biologique tel que le collagène. Dans le cas où le corail est remplacé par du collagène, réticulé ou non, ce dernier peut servir également à la fois comme support et comme matrice favorisant la gélification biologique pour la constitution du réseau de cellules.

Selon un mode de réalisation avantageux de l'invention, le corail à haute porosité utilisé est de type sphérique.

Selon un autre mode de réalisation de l'invention, l'implant comporte un support d'origine biologique formant une matrice solide, ledit support contenant :
- du collagène plus ou moins réticulé par exemple sous forme de fibres ou d'éponges telles que commercialisées sous les marques Hémostagène® ou Paroguide® ;
- de l'os en poudre ou en fragment ou ;
- des polymères à base d'hydrates de carbones tels que le dextran ou l'acide hyaluronique.

Selon un mode de réalisation avantageux de l'invention, l'armature solide du support d'origine biologique est susceptible de résorption in vivo.

L'implant ainsi constitué permet la formation in vivo d'un neo-organe individualisé et stable si nécessaire, dans lequel le support biologique est susceptible d'être progressivement résorbé en laissant place à une structure comportant un tissu conjonctif vascularisé de forme relativement comparable à celle de l'implant, ce néo-organe ayant cependant le cas échéant un volume diminué par rapport au volume de l'implant d'origine.

Un implant particulièrement préféré dans le cadre de l'invention est tel que les constituants capables d'induire et/ou de favoriser la gélification des cellules sont par exemple du collagène non réticulé ou des alginates.

En particulier, on aura recours à du collagène par exemple de type I, notamment à une concentration de 1,5 mg/ml.

D'autres constituants pour gélifier les cellules peuvent être naturellement utilisés dès lors qu'ils ont la propriété fonctionnelle recherchée et qu'ils présentent les propriétés de biocompatibilité requises pour être introduits in vivo chez l'homme ou chez l'animal.

### Utilisation de l'implant de l'invention

Un implant particulier propre à la réalisation de l'invention est en outre caractérisé en ce que les cellules sont des cellules recombinées comportant une information génétique étrangère à leur génome, susceptibles d'exprimer cette information supplémentaire in vivo et ayant la capacité d'être tolérées immunologiquement par un organisme auquel elles seraient administrées.

De manière préférée, un implant selon l'invention est caractérisé en ce que les cellules recombinantes sont modifiées par un vecteur contenant une séquence ou plusieurs séquences de nucléotides exogène(s), codant pour un antigène ou un déterminant antigénique ou codant pour un polypeptide ou une glycoprotéine, soluble dans le sérum, par exemple pour un polypeptide ou une glycoprotéine d'intérêt thérapeutique, notamment pour une hormone, une protéine ou une glycoprotéine de structure ou une protéine ou une glycoprotéine du métabolisme ou une protéine ou une glycoprotéine virale ou une protéine ayant les caractéristiques d'un anticorps ou d'un fragment d'anticorps.

De façon préférée, l'implant contient en outre un ou plusieurs facteurs angiogéniques, en particulier du bFGF, préférablement incorporés lors de la mise en contact du support biocompatible avec les cellules et le constituant capable d'induire et/ou de favoriser leur gélification.

La fixation du facteur angiogénique, par exemple du bFGF, est favorisée par la présence de l'agent de gélification.

Cet agent contribue à la fixation stable des facteurs angiogéniques au support d'origine biologique de l'implant.

De façon particulièrement avantageuse, l'implant contient également de l'héparine ou un dérivé héparinique tel que l'héparan sulfate ou des fractions hépariniques.

L'héparine et ses dérivés sont capables de se fixer aux constituants favorisant la gélification et ainsi d'augmenter l'affinité des facteurs angiogéniques aux constituants permettant la gélification. En particulier, l'héparine se fixe au collagène et augmente ainsi l'affinité du bFGF pour le collagène.

L'implant selon l'invention peut être mis en oeuvre de façon permanente ou transitoire, pour l'utilisation chez l'homme ou chez l'animal. En effet, sa capacité à constituer in vivo un néo-organe individualisé, permet de le retirer par prélèvement si nécessaire.

Un implant selon l'invention est, de façon particulièrement intéressante, utilisable pour :
- le traitement de maladies génétiques, en particulier pour le traitement de maladies de surcharge lysosomale, de l'hémophilie A ou de l'hémophilie B, de la β-thalassémie, la séquence de nucléotides exogène contenue dans les cellules recombinantes correspondant respectivement à celles qui codent pour la β-glucuronidase pour le facteur VIII, le facteur IX ou l'érythropoïétine, ou pour une partie active de ces séquences ;
- le traitement de maladies acquises, par exemple pour le traitement de maladies virales, notamment pour le traitement d'une infection due au rétrovirus HIV par exemple par l'expression et la sécrétion dans le sérum de molécules CD4 solubles ou d'une protéine anti-virale soluble ;
- le traitement de tumeurs, la séquence de nucléotides exogène contenue dans les cellules recombinantes codant pour un composé capable de favoriser ou d'augmenter la réponse immunitaire contre les cellules de la tumeur.

L'invention a aussi pour objet une composition caractérisée en ce qu'elle comprend un implant selon l'invention et un ou plusieurs autres constituants tels que par exemple un antigène, un adjuvant notamment pour la vaccination.

Les moyens décrits dans l'invention permettent donc d'envisager le traitement de maladies génétiques ou acquises sur une période longue, supérieure à plusieurs mois, et ce sans administration répétée du produit d'expression de la séquence de nucléotides exogène dont on recherche l'activité thérapeutique. De plus, les moyens décrits dans l'invention permettent l'obtention sous forme de protéine sécrétée d'une quantité de produit d'expression de la séquence de nucléotides exogène, suffisante pour avoir un effet thérapeutique in vivo.

Une autre application des cellules recombinantes selon l'invention ou des compositions ou implants les contenant, concerne la préparation d'anticorps contre le produit d'expression de la séquence de nucléotides exogène, contenu dans le vecteur introduit dans les cellules recombinantes. De préférence, ces anticorps sont obtenus in vivo lorsque les cellules recombinantes sont implantées dans un organisme.

Cette utilisation permet par exemple de produire des anticorps contre un antigène dont on n'aurait pas identifié la séquence en acides aminés ni nécessairement séquencé l'ADN ou l'ADNc. Selon l'effet recherché, l'introduction du vecteur recombinant selon l'invention, sera effectuée dans un type cellulaire défini.

De même, l'invention permet la production in vivo d'anticorps contre un antigène déterminé, par exemple à des fins de vaccination d'un patient.

Des cellules recombinantes telles que décrites plus bas, ou des compositions ou implants les contenant peuvent également être mises en oeuvre pour le traitement de tumeurs, la séquence de nucléotides exogène contenue dans les cellules recombinantes codant pour une substance capable de favoriser ou d'augmenter la réponse immunitaire contre les cellules de la tumeur.

A titre particulier, les cellules recombinantes utilisables pour le traitement de tumeurs, peuvent être des cellules obtenues par recombinaison de cellules de tumeurs, prélevées chez un patient, avec un vecteur rétroviral répondant aux définitions données dans les pages suivantes.

### Kit selon l'invention

L'invention comprend également un kit pour la préparation d'un implant pour réaliser in vivo l'expression et la sécrétion par des cellules d'un composé pour produire un effet thérapeutique recherché. Le kit de l'invention comprend généralement :
- un support biocompatible permettant l'ancrage biologique desdites cellules ; et
- un constituant capable d'induire et/ou de favoriser la gélification desdites cellules.

Particulièrement, le support biocompatible est un support d'origine biologique à base de calcaire, en particulier de carbonate de calcium, de préférence de corail. Le constituant capable d'induire et/ou de favoriser la gélification des cellules est de préférence du collagène, en particulier du collagène de type I, de préférence à une concentration de l'ordre de 1,5 mg/ml.

Le kit selon l'invention peut également inclure un ADN comprenant une séquence codant pour le composé exprimé et secrété par lesdites cellules. Cet ADN peut être utilisé pour transformer les cellules prélevées chez le patient à traiter.

Plus particulièrement, l'ADN faisant partie du kit est un vecteur rétroviral tel que décrit plus loin.

Le kit peut aussi comprendre des cellules ayant la capacité d'exprimer et de secréter naturellement ou après recombinaison un composé déterminé, par exemple un composé ayant un intérêt thérapeutique.

### Vecteur rétroviral et cellules recombinantes

### a) Vecteur rétroviral

Un nouveau vecteur rétroviral recombinant selon l'invention est caractérisé en ce qu'il comprend :
. une séquence d'ADN de provirus modifiée en ce que :
   - les gènes gag, pol et env ont été délétés au moins en partie et ce afin d'obtenir un ADN proviral incapable de se répliquer, cet ADN ne pouvant, en outre, être recombiné pour former un virus sauvage ;
   - la séquence LTR comporte une délétion dans la séquence U3, telle que la transcription sous forme d'ARNm qu'elle contrôle est réduite de manière significative, par exemple d'au moins 10 fois, et
   - ce vecteur rétroviral recombinant comprenant, en outre, une séquence de nucléotides exogène sous le contrôle d'un promoteur, par exemple d'un promoteur constitutif ou inductible, exogène.

Les éléments principaux formant ce vecteur sont décrits ci-dessous de façon détaillée.

### 1) ADN de provirus

Une séquence d'ADN de provirus ou "séquence d'ADN proviral" est une séquence d'ADN transcrite à partir de l'ARN génomique du virus lorsqu'il est intégré dans les cellules hôtes du virus. L'ADN du provirus comprend ainsi des séquences codant pour les protéines gag, pol et env du rétrovirus, qui correspondent respectivement aux nucléoprotéines, polymérases, protéines et glycoprotéines d'enveloppe.

Le vecteur rétroviral selon l'invention est tel que les gènes gag, pol et env de l'ADN du provirus ont été délétés au moins en partie et ce afin d'obtenir un ADN proviral incapable de se répliquer, cet ADN ne pouvant, en outre, être muté de façon réverse ou recombiné pour former un virus sauvage.

L'ADN du provirus comporte également des enchaînements appelés LTR (Long Terminal Repeat), qui comportent des régions appelées R, U3 et U5. Ces séquences de la région LTR interviennent dans le cycle de réplication du rétrovirus.

La séquence LTR de l'ADN proviral a également été mutée par délétion, par exemple dans sa partie U3 ; cette délétion affecte les fonctions de l'activateur interne de la région LTR. De plus, cette délétion permet de diminuer et de préférence, de neutraliser l'effet transcriptionnel de la région LTR, en conservant la capacité du promoteur exogène de promouvoir et contrôler l'expression de la séquence de nucléotides exogène contenue dans le vecteur rétroviral.

On peut aussi envisager la délétion de l'ensemble de la séquence constituant le promoteur et l'activateur. Toutefois, une telle délétion présente l'inconvénient d'être accompagnée d'une diminution importante des titres du vecteur.

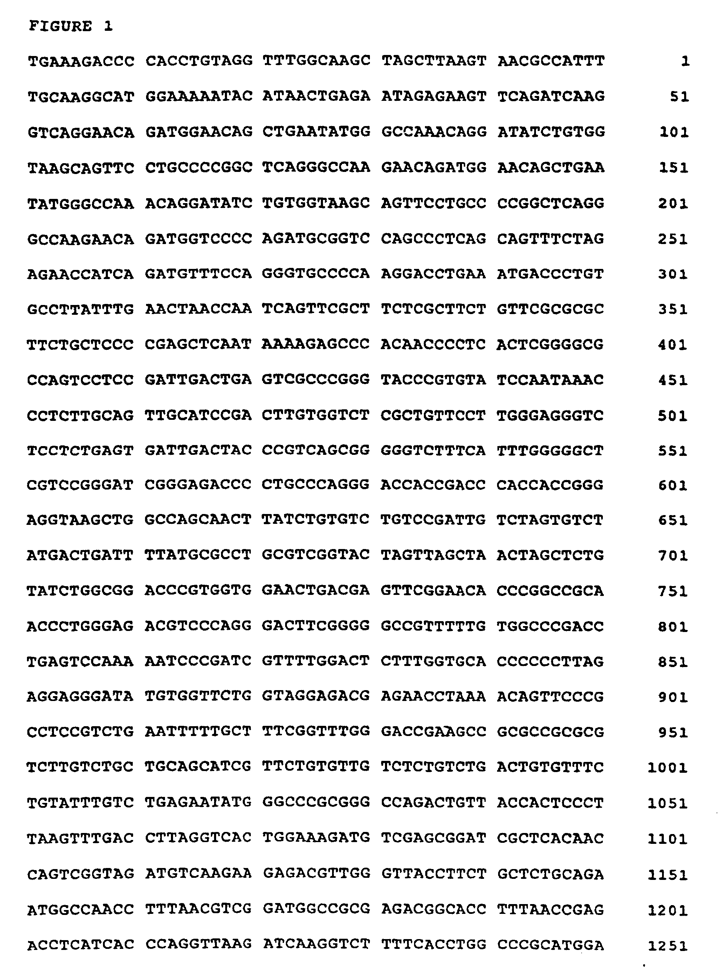

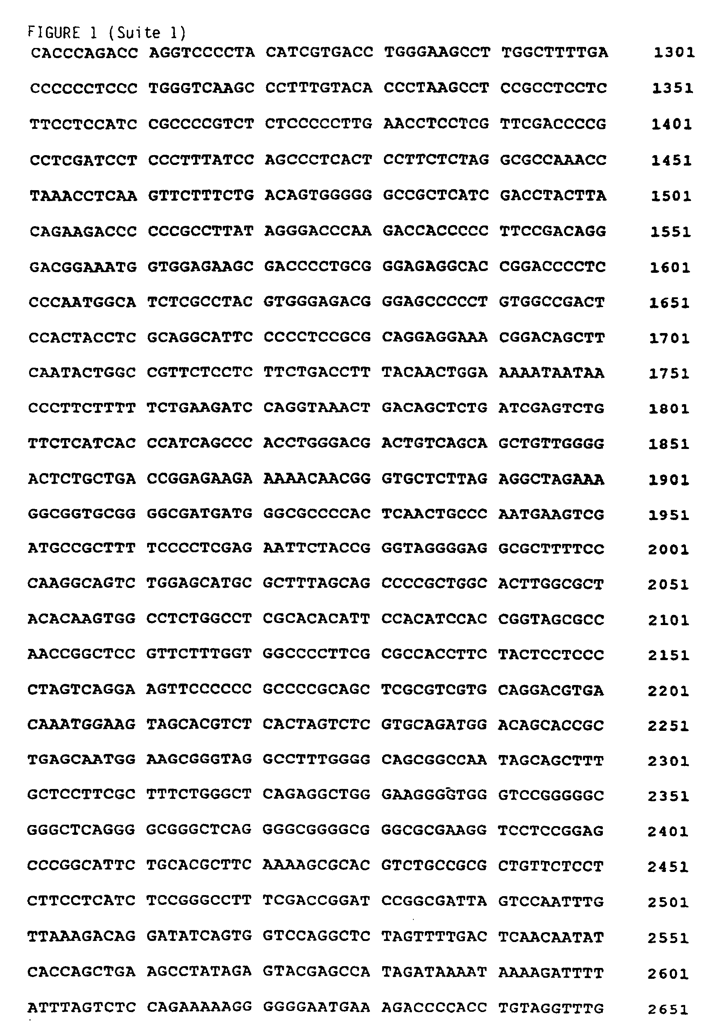

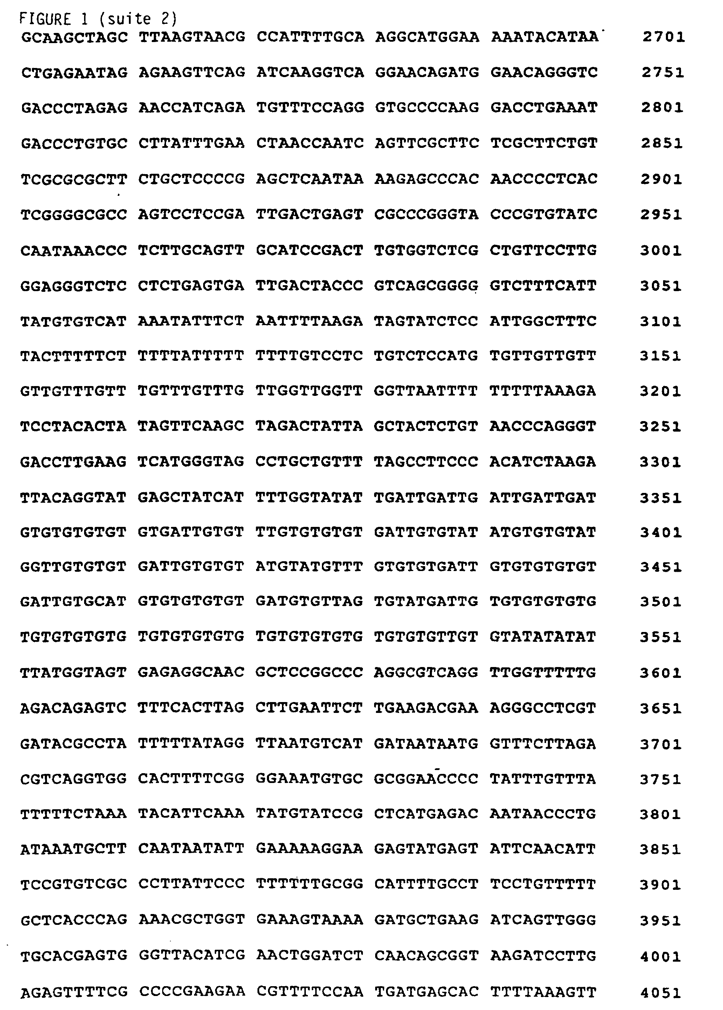

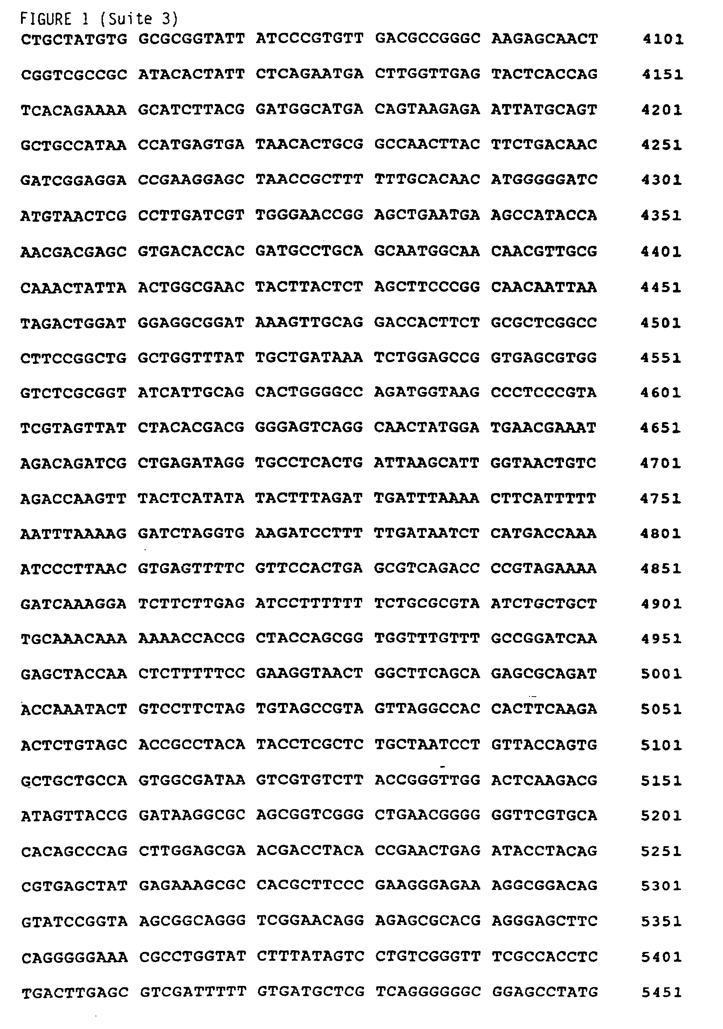

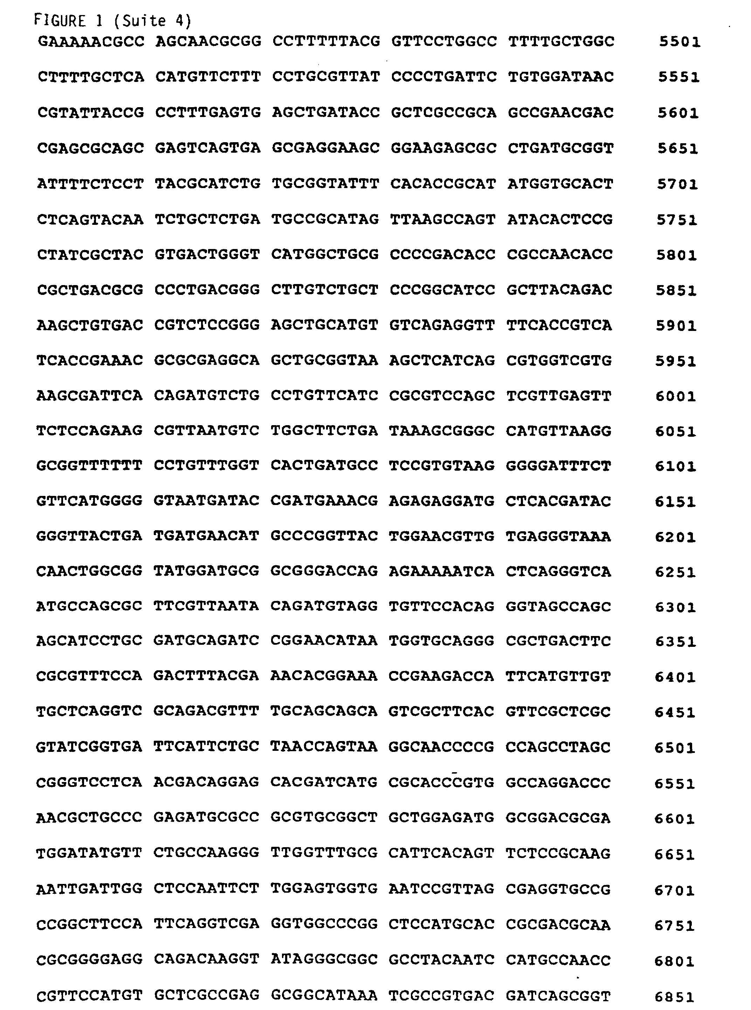

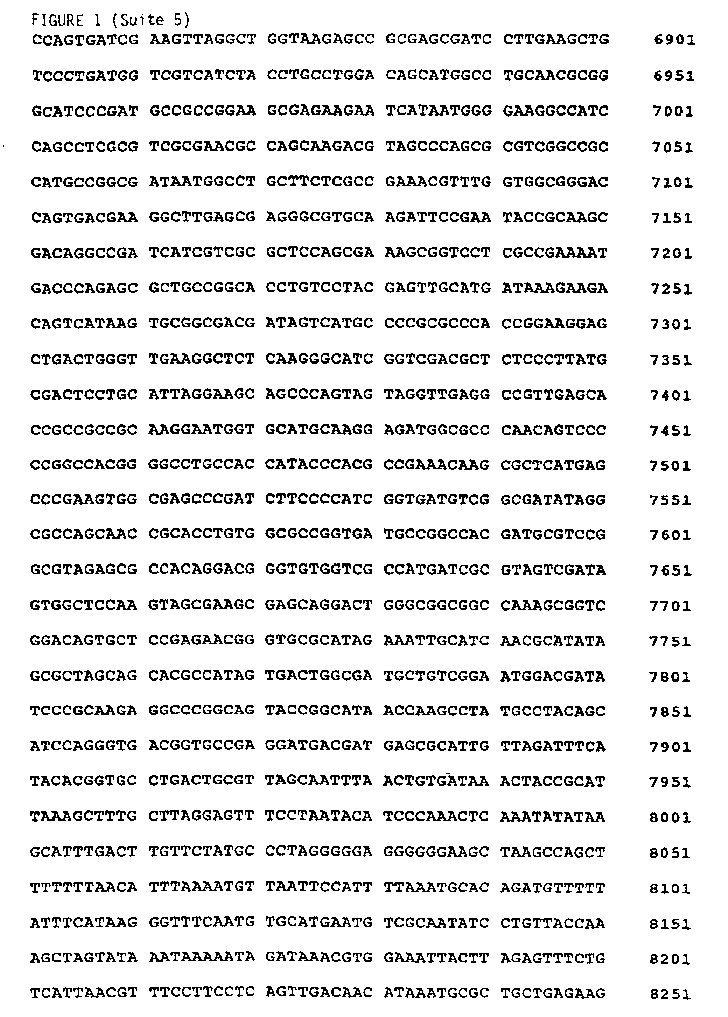

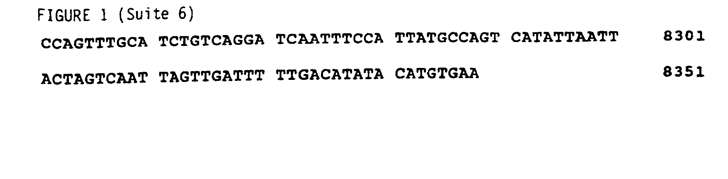

Selon une variante de réalisation de l'invention, la séquence provirale en amont du promoteur exogène est la séquence de nucléotides située entre les nucléotides 1 et 1500 environ selon la numérotation de la séquence représentée à la figure 1. Cette séquence en amont du promoteur est essentiellement dépourvue de l'ensemble des gènes gag, pol et env de la séquence provirale.

La séquence de nucléotides exogène est avantageusement insérée sous le contrôle du promoteur exogène, à la place des séquences gag, pol et env délétées.

Lorsque le promoteur est le promoteur PGK-1 décrit plus loin, la séquence de nucléotides exogène est avantageusement insérée au site BamHI en aval de ce promoteur.

### 2) Séquence de nucléotides exogène

Une "séquence de nucléotides exogène" selon l'invention est une séquence qui n'est pas exprimée naturellement dans une cellule dans laquelle on introduit le vecteur rétroviral de l'invention, ou y est exprimée de façon insuffisante, ou dont on veut obtenir une expression plus importante que l'expression normale. Ce défaut d'expression ou cette insuffisante d'expression résultent de la nature de la cellule ou survient à cause d'une pathologie affectant cette cellule chez un individu donné.

De façon préférée, la "séquence de nucléotides exogène" code pour un polypeptide déterminé. Par "polypeptide", on entend toute séquence d'acides aminés, quelle que soit sa taille, cette expression comprenant les protéines et les peptides. Le polypeptide peut être sous forme glycosylée ou non glycosylée.

### 3) Promoteur exogène

Le promoteur utilisé pour la construction de rétrovirus recombinants selon l'invention est avantageusement exogène par rapport à la séquence dont il contrôle l'expression en ce sens qu'il ne lui est pas naturellement associé. On peut aussi utiliser le promoteur du gène exogène transféré.

Le promoteur utilisé dans le vecteur de l'invention peut être de type constitutif ou inductible. Ce promoteur exogène contrôle l'expression de la séquence de nucléotides exogène. Le promoteur peut, le cas échéant, être accompagné d'une séquence nucléique régulatrice par exemple activatrice ("enhancer") qui servirait à réguler son activité.

Dans le cas de promoteurs inductibles, des promoteurs tels le promoteur Mx de souris, les promoteurs comprenant un opérateur tétracycline ou encore des promoteurs régulés par des hormones, en particulier des stéroïdes, peuvent être utilisés. En ce qui concerne les promoteurs dits "constitutifs", l'utilisation de promoteurs internes actifs dans les fibroblastes au repos, tels le promoteur PGK ou un autre promoteur de gène de ménage est préférée.

De façon particulièrement avantageuse pour la réalisation d'un vecteur rétroviral selon l'invention, le promoteur constitutif exogène est un promoteur sans TATA box et en particulier le promoteur du gène de la phosphoglycérate kinase (PGK-1). Ce promoteur est soit le promoteur de souris ou le promoteur humain tel que décrit par Adra et al (Gene 60, 1987, 65-74).

L'insertion du promoteur PGK-1 dans un vecteur rétroviral permet le transfert du gène dans des fibroblastes cutanés, par exemple, et l'expression stable à haut niveau et à très long terme de ce gène après que les fibroblastes aient été implantés in vivo chez un receveur, par exemple un mammmifère.

D'autres promoteurs constitutifs peuvent être utilisés à la place du promoteur PGK-1. De façon générale, on aura recours à un promoteur actif dans les cellules que l'on cherche à transformer avec le vecteur rétroviral et en particulier des promoteurs actifs dans des fibroblastes à l'état quiescent, par exemple des promoteurs précédant les gènes du cytosquelette. On peut également citer le promoteur de la β-actine. (Kort et al., 1983, Nucl. Acides Res. 11: 8287-8301) ou le promoteur du gène de la vimentine (Rettlez et Basenga (1987), Mol. Cell. Biol. 7: 1676-1685). Le promoteur utilisé peut être dépourvu de "TATA box".

### 4) Réalisations préférentielles du vecteur rétroviral

Un premier vecteur rétroviral particulier dans le cadre de l'invention est tel qu'une séquence de nucléotides exogène et un promoteur constitutif exogène, ainsi que la séquence de l'ADN proviral, sont portés par un plasmide. A titre d'exemple, ces séquences peuvent être portées par le plasmide pBR322 qui permet l'introduction de l'ADN dans les lignées cellulaires dans lesquelles on cherche à produire le vecteur.

Selon un autre mode de réalisation avantageux de l'invention, un vecteur répondant aux définitions précédentes est en outre caractérisé en ce que l'ADN proviral est issu du rétrovirus Mo-MuLV. D'autres rétrovirus de la famille des MuLV peuvent être utilisés et l'on citera par exemple les rétrovirus HaSV ou F-MuLV.

De préférence, les séquences des gènes pol et env de l'ADN proviral sont totalement délétées. Dans ce cas, la séquence du gène gag peut être également entièrement délétée ou au contraire être conservée en partie, dès lors que l'ADN du provirus ainsi constitué n'est plus susceptible de se répliquer.

Avantageusement, un vecteur rétroviral tel que défini précédemment est caractérisé en ce que la région U3 du fragment LTR3' est délétée au niveau du nucléotide 2797 de la figure 1. Cette délétion correspond à une délétion d'un fragment situé entre les nucléotides 7935 et 8113 selon la numérotation de la séquence publiée par Shinnick et al (Nature 293, 543-548, 1981). Ce fragment délété de la séquence U3 du LTR3' comporte la région activatrice du LTR.

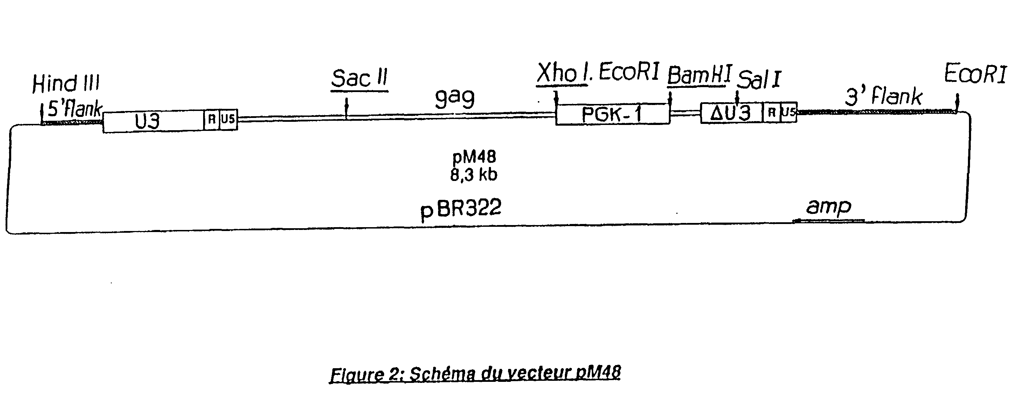

A titre préféré, un vecteur rétroviral selon l'invention est le vecteur de type pM48 dérivé du virus de Moloney dans lequel l' "enhancer" viral localisé en V3 a été supprimé et comportant un promoteur interne actif dans les fibroblastes au repos, de préférence le promoteur PGK ou un autre promoteur de gène de ménage, tel que le vecteur représenté à la figure 2, modifié par la séquence de nucléotides exogène au site BamHI.

Le vecteur rétroviral M48 LacZ (encore désigné par l'expression pM48 LacZ) a été déposé à la CNCM (Collection Nationale de Culture de Microorganismes, Paris - France) sous le N° I-1298, le 16 Avril 1993.

Ce vecteur est dérivé du vecteur pM48 et est caractérisé en ce qu'il contient en aval du promoteur constitutif exogène, un fragment BamHI du gène de la β-galactosidase.

Ce fragment du gène de la β-galactodidase peut aisément être délété et remplacé par une séquence de nucléotides exogène d'intérêt, par exemple une séquence codant pour une protéine ou une glycoprotéine susceptible d'avoir un intérêt thérapeutique ou contre le produit protéique de laquelle on souhaiterait par exemple obtenir des anticorps.

Un vecteur rétroviral de l'invention peut également contenir une séquence capable d'augmenter l'activité transcriptionnelle du promoteur, soit de manière constitutive, soit de manière inductible. Le vecteur peut contenir, en amont du promoteur exogène, une séquence d'activateur.

### b) Cellules recombinantes

L'invention concerne par ailleurs des cellules recombinantes, caractérisées en ce qu'il s'agit de cellules ayant la capacité d'être tolérées immunologiquement par un organisme chez lequel elles seraient implantées, modifiées par un vecteur rétroviral répondant à l'une des définitions précédentes.

De telles cellules, peuvent être des cellules issues de l' organisme chez lequel elles doivent être implantées après recombinaison par transduction avec le vecteur de l'invention, ou des cellules dépourvues à leur surface d'antigènes reconnus par le système immunitaire de l'organisme chez lequel elles sont implantées.

Il peut également s'agir de cellules endothéliales, de myoblastes, de cellules musculaires ou de cellules tumorales irradiées ou traitées selon d'autres procédés pour éviter leur prolifération et prélevées chez un patient et dont on souhaite modifier le patrimoine génétique afin qu'elles puissent intervenir sur le développement de la tumeur.

A titre préféré, ces cellules recombinantes sont des fibroblastes recombinants et en particulier des fibroblastes cutanés. De préférence, il s'agit de fibroblastes autologues par rapport au patient chez lequel on souhaite les implanter après leur modification par un vecteur selon l'invention. Des fibroblastes préparés à partir d'autres organes peuvent être utilisés, comme par exemple des fibroblastes isolés à partir d'un cordon ombilical. Les fibroblastes génétiquement modifiés et utilisés dans le contexte de la présente invention sont des fibroblastes non immortalisés.

Ces cellules peuvent être modifiées par un vecteur recombinant codant pour un polypeptide déterminé, ledit vecteur permettant l'expression de l'ADN étranger dans les cellules. Elles peuvent aussi être modifiées par les méthodes dites de recombinaison homologue. La pénétration du vecteur dans les cellules est réalisée en utilisant l'électroporation ou le précipité au phosphate de calcium ou encore par toute autre méthode impliquant l'entrée d'un acide nucléique soit seul soit par l'intermédiaire d'un virus recombinant par exemple.

Par "cellules recombinées", on entend également des cellules autologues tumorales irradiées préalablement à leur introduction dans le gel, ayant la capacité de maintenir à leur surface des antigènes tumoraux accessibles au système immunitaire de l'hôte chez lequel l'implant selon l'invention a été introduit.

Les cellules recombinantes de l'invention sont également obtenues soit par l'infection des cellules à modifier avec un vecteur rétroviral de l'invention, soit par d'autres méthodes de transduction ADN nu, complexe ADN-protéines ou vecteur adénovirus. Dans le cas d'une infection avec un vecteur rétroviral, la séquence codant pour le polypeptide dont on recherche l'expression (séquence de nucléotides exogène) est introduite dans l'ADN proviral.

De manière préférée, les cellules recombinantes sont modifiées par un vecteur contenant une séquence ou plusieurs séquences de nucléotides exogène(s), codant pour un antigène ou un déterminant antigénique ou codant pour un polypeptide ou une glycoprotéine, soluble dans le sérum, par exemple pour un polypeptide ou une glycoprotéine d'intérêt thérapeutique, notamment pour une hormone, une protéine ou une glycoprotéine de structure ou une protéine ou une glycoprotéine du métabolisme ou une protéine ou une glycoprotéine virale ou une protéine ayant les caractéristiques d'un anticorps ou d'un fragment d'anticorps.

A titre d'exemple, cette séquence de nucléotides exogène code pour la β-glucuronidase, ou une autre enzyme lysosomale telle que l'α-iduronidase ou l'arylsulfatase B, un facteur de coagulation tel que le facteur VIII ou le facteur IX, l'érythropoïétine, ou toute partie active de l'une de ces protéines.

D'autres avantages et caractéristiques de l'invention apparaissent dans les exemples et les figures qui suivent.
- Figure 1 : séquence de nucléotides du vecteur M48 ;
- Figure 2 : représentation du vecteur PM48.

### EXEMPLES

### PREPARATION DU VECTEUR RECOMBINANT

Le transfert et l'expression de matériel génétique exogène permet de modifier expérimentalement les propriétés d'un tissu. Des traitements reposant sur ce principe pourraient être proposés à des malades présentant des déficiences génétiques ou des maladies acquises. L'exemple décrit ci-dessous fait appel à un vecteur rétroviral pour introduire dans des cellules l'information génétique codant pour une protéine sécrétée. La réimplantation dans l'organisme des cellules génétiquement modifiées est réalisée en associant ces cellules avec du collagène, un facteur angiogénique et l'armature de type corail ou collagène réticulé. L'expression in vivo à long terme du matériel génétique ainsi transféré est obtenue en utilisant le promoteur du gène murin de la phosphoglycérate kinase.

### 1 - VECTEUR RETROVIRAL

### 1.1 - Caractéristiques

Le vecteur rétroviral M48 (figure 2) est construit à partir de séquences provirales isolées de l'ADN génomique de cellules de rat infectées par le rétrovirus leucémogène murin de Moloney (Mo-MuLV). Les gènes gag, pol et env qui codent pour les protéines du virus ont été retirés et remplacés par les séquences à véhiculer. On a conservé les séquences rétrovirales nécessaires en cis à la production et à la maturation des transcrits du génome viral recombinant, à leur empaquetage dans des particules virales infectieuses, à leur transcription inverse et à leur intégration dans le génome de la cellule infectée. Les séquences du provirus recombinant et les fragments flanquants d'ADN cellulaire sont portés par le plasmide bactérien pBR322.

Le promoteur du gène de la phosphoglycérate kinase (PGK-1) de souris a été inséré à la place des gènes viraux. Un site de clonage unique BamHI permet de placer les séquences à véhiculer dans le vecteur rétroviral sous le contrôle de ce promoteur.

Ce vecteur rétroviral comporte une délétion dans les séquences U3 du LTR interne (Cone et al., Mol. Cel. Biol. 7, 887-897, 1987). Ceci entraîne, dans la cellule infectée, une forte diminution des ARNm démarrant au niveau du LTR 5', et une prédominance des ARNm produits à partir du promoteur PGK-1. L'utilisation de ce vecteur résulte donc en l'insertion, dans le génome de la cellule cible d'une cassette d'expression de la séquence d'intérêt sous le contrôle du promoteur PGK-1.

### 1.2. - Structure du vecteur

La séquence complète du vecteur M48 (Figure 1) comprend :
- **Position 1 à 2019 :** Séquences du Mo-MuLV comprenant la longue répétition terminale 5'(ou 5'LTR ou LTR5'), les séquences d'empaquetage Ψ+ qui incluent une partie du gène gag (position -447 à 1564 dans la numérotation de Shinnick et al. (Nature **293**, 543-548, 1981). Ces séquences sont modifiées de la façon suivante : insertion d'un linker Sac II (5'CCCGCGGG3') en position 1074 (position 626, Shinnick et al.).
   Ceci conduit à une mutation en phase avec la séquence codant pour gag.
- **Position 2020 à 2526 :** Promoteur du gène de souris codant pour la phosphoglycérate kinase (position -524 à -20, avant le codon de début de traduction, d'après Adra et al. Gene **60,** 65-74, 1987).
- **Position 2527 à 2532 :** Site unique BamHI pour l'insertion de séquences qui seront exprimées sous le contrôle du promoteur PGK-1.
- **Position 2533 à 3101 :** Séquences du Mo-MuLV comprenant l'extrémité 3' du gène env (32 codons) et le LTR3'. Ces séquences sont modifiées de la façon suivante : délétion des séquences U3 du LTR comprises entre 7935 et 8113 dans la numérotation de Shinnick et al. et insertion d'un linker Sal I (2798 dans la numérotation présente).
- **Position 3102 à 3674 :** Séquences génomiques de rat.
- **Position 3675 à 8003 :** Séquences du plasmide vecteur pBR322 (position 4363 à 29) comprenant le gène de β-lactamase. Ces séquences sont modifiées de la façon suivante : destruction du site BamH I (position 7657, numérotation présente).
- **Position 8004 à 8388 :** Séquences génomiques de rat.

### 1.3. - Production de vecteur rétroviral

Cette production s'effectue en introduisant la structure provirale recombinante dans une lignée cellulaire où les gènes gag, pol et env sont exprimés constitutivement.

Cette lignée, dite transcompl ementante ou d'empaquetage synthétise des particules rétrovirales dépourvues d'ARN génomique. La lignée ΨCRIP, dérivée de fibroblastes de souris NIH/3T3 est utilisée ici (Danos et Mulligan Proc. Natl. Acad. Sci (USA) **85**, 6460-6465, 1988). Lorsque la construction recombinante est introduite par transfection, les ARN transcrits à partir du LTR5' sont encapsidés et les cellules produisent alors des particules capables de transmettre le génome recombinant mais incapables de se répliquer.

Après transfection de la construction recombinante, les cellules productrices de vecteur rétroviral sont sélectionnées. On utilise pour la préparation de vecteur, soit un clone producteur de hauts titres viraux, soit une population polyclonale. Les étapes de l'isolement d'un clone producteur sont les suivantes :
1) Les cellules ΨCRIP sont transfectées par un coprécipité au phosphate de calcium (Graham et Van Der Erb, Virology, **52,** 456, 1973) de la construction rétrovirale et du plasmide pSV2neo (Southern and Berg, J. Mol. Appl. Gen. **1**, 327-341, 1982), dans un rapport molaire de 10 pour 1.
2) Les cellules ayant intégré de manière stable et exprimant l'ADN exogène sont sélectionnées en présence de G418 à 1 mg/ml (Geneticin, Gibco).
3) La population de cellules résistantes au G418 où des clones individuels isolés sont testés pour leur capacité à produire le vecteur rétroviral. Pour cela, des cellules cibles NIH/3T3 sont mises en présence du milieu de culture recueilli à partir de cellules ΨCRIP, et la transmission du provirus recombinant est analysée après 48 heures. L'analyse, effectuée à partir de l'ADN génomique des cellules cibles, se fait par PCR (méthode qualitative) ou par Southern blot (méthode quantitative).
4) Une banque de cellules productrices est conservée à - 135°C.

### 2 - PREPARATION DES STOCKS DE VECTEUR RETROVIRAL

Le surnageant de culture des cellules d'empaquetage est recueilli après une incubation de 24 heures sur les cellules d'empaquetage produisant le vecteur rétroviral, et mis en contact avec les cellules cibles pour réaliser l'infection.

### 2.1. - Préparation en masse

Un stock d'une centaine d'ampoules contenant des cellules productrices est conservé à -135°C. Après décongélation, les cellules sont amplifiées pendant 10 jours, successivement en flacon puis en rollers de 875 cm², jusqu'à obtention de 20 rollers confluents. La production est alors commencée. Elle dure 4 ou 5 jours. Le milieu de culture est du DMEM contenant 1 g/l de glucose, 10 mM de pyruvate de Na, et additionné avec 5 % de sérum de veau nouveau-né (Hyclone). Chaque roller permet le conditionnement de 100 ml de milieu par 24 heures. Ce surnageant de culture est centrifugé pour éliminer les débris cellulaires. La production hebdomadaire est de 8 à 10 litres de surnageant viral.

### 2.2. - Concentration

Le surnageant peut être concentré pour augmenter le titre infectieux du vecteur rétroviral. Après centrifugation, le surnageant est dialysé tangentiellement contre une membrane Sartocon de porosité 100.000, en utilisant le dispositif Crossflow de Sartorius. Une concentration de 20 fois est obtenue en 3 heures. Elle s'accompagne d'une augmentation de 20 fois des titres infectieux. Après concentration, la préparation virale est utilisée immédiatement, ou conservée à -80°C si le délai d'utilisation est supérieur à 2 semaines.

### 3 - FIBROBLASTES CUTANES

### 3.1. - Isolement et mise en culture

Les fibroblastes destinés au transfert de gène sont obtenus par une biopsie cutanée effectuée en conditions d'aseptie très soigneuse. Chez la souris, un animal syngénique est sacrifié à cet usage. Chez le gros mammifère, un ou plusieurs fragments de peau d'environ 10 cm² sont prélevés au cours d'une anesthésie générale. Après découpage en lambeaux de quelques mm² , les fragments sont dissociés par un traitement enzymatique. Pour un fragment de 10cm² , la réaction est effectuée pendant 2 heures à 37°C en agitation douce dans 50ml de milieu RPMI 1640 additionné de 10 % de sérum de veau foetal, 100 mg de collagénase (Worthington), 500 U de dispase (Collaborative Research Inc.). Après centrifugation et lavage, les cellules sont comptées, puis ensemencées dans des flacons de 150 cm² à la concentration de 1 à 5x10⁶/cm2. Le milieu de culture est du RPMI 1640 additionné de 10 à 20 % de sérum de veau foetal.

### 3.2. - Infection par le vecteur rétroviral

Les infections avec la préparation de vecteur rétroviral sont débutées le lendemain de la mise en culture, alors que la densité cellulaire est inférieure à 20 % de confluence. Elles sont répétées quotidiennement, suivant le même protocole pendant 4 ou 5 jours. Après retrait du milieu de culture, les cellules sont mises en contact avec la préparation de vecteur rétroviral non concentrée ou concentrée, dans tous les cas, additionnée avec 8µg/ml de polybrene. L'incubation est effectuée pendant 2 heures à 37°C, puis, sans lavage préalable, la préparation virale est remplacée par du milieu de culture.

Lorsqu'un vecteur codant pour une protéine détectable à l'examen microscopique est utilisé, on constate que ce procédé permet l'introduction du gène étranger dans plus de 90 % de fibroblastes. Le niveau de sécrétion de la protéine dans le surnageant de culture peut être contrôlé au cours et à la fin de ce processus. Les cellules peuvent être congelées en partie ou en totalité à ce stade.

Après infection, les cellules sont amplifiées jusqu'à obtention d'un nombre suffisant pour la réimplantation, soit 2-6x10⁸ cellules/kg de poids corporel. L'amplification est réalisée dans des plateaux de culture (multitray, Nunc).

### 3.3. - Recueil des cellules génétiquement modifiées.

Après amplification, les cellules sont recueillies par traitement à la trypsine. Après lavage et comptage, elles sont disponibles pour servir à la formation d'un neo-organe. Un échantillon est conservé pour contrôler le transfert génétique par Southern blot, ainsi que l'expression et la sécrétion de la protéine étrangère par une technique appropriée.

### Exemple 1 :

**Secrétion d'une enzyme lysosomale (β-glucuronidase) chez la souris avec des implants contenant des fibres de PTFE, du collagène de queue de rat et des fibroblastes cutanés modifiés avec un vecteur rétroviral.**

### 1.1. - Constituants

Trois composants sont assemblés in vitro. La méthode décrite ci-dessous est applicable à la réalisation de néo-organes de 1 ml contenant 5x10⁶ à 10⁷ cellules génétiquement modifiées. Les mêmes proportions sont utilisées pour la formation de néo-organes de grande taille contenant 1 à 5x10⁹ cellules.
1) Des fibres de polytetrafluoroéthylène (Gore et Associates) stérilisées par autoclavage (120°C, 30 minutes) sont recouvertes par du collagène de type 1. Les fibres sont baignées dans une solution de collagène de queue de rat (Sigma) à 0.1 % dans de l'acide acétique 0.1N. Ce traitement s'effectue sous vide pendant 1 heure à température ambiante, afin de chasser l'air présent entre les fibres synthétiques. Les collagènes d'origine murine, bovine ou humaine peuvent être indifféremment utilisés. Après séchage pendant 24 heures sous une hotte à flux luminaire, les fibres ainsi entourées d'une fine pellicule de collagène, sont incubées en présence d'un facteur de croissance angiogénique (FGF basique, Farmitalia, 10-20 ng/50 mg de fibres dans une solution de PBS-5% β-mercaptoethanol). Les fibres ainsi traitées, sont lavées en PBS et conservées à 4°C.
2) Une solution constituée des éléments suivants est préparée. Pour 1 ml : RPMI 1640 10x (Gibco), 100 µl ; bicarbonate 7,5 %, 12 µl ; NaOH 7,5 N, 8 µl ; Hepes 1 M, 2,5 µl ; Penicilline/Glutamine (Gibco), 100 µg ; bFGF (Farmitalia) 10 ng ; collagène (Bioethica) 1,5 mg ; eau distillée qsp 1 ml. Cette solution de consistance sirupeuse, est conservée à + 4°C.
3) Après lavage, les cellules génétiquement modifiées sont resuspendues dans 100 µl de RPMI 1640 avec 15 % de sérum de veau foetal.

### 1.2. - Assemblage

L'assemblage in vitro du néo-organe s'effectue stérilement sous hotte, de la manière suivante :
1) 1 mg de fibres traitées sont étalées dans une boîte de culture (plaque Corning 24 puits).
2) Les cellules génétiquement modifiées (100µl) sont incorporées à la solution de collagène (1ml), à l'aide d'un agitateur,
3) Ce mélange est déposé sur les fibres.

L'ensemble est placé dans un incubateur (37°C, 5 % CO₂) pendant 30 minutes afin d'obtenir la gélification du collagène. Lorsque celle-ci survient, 1ml de RPMI 1640 avec sérum de veau foetal est déposé sur le gel afin de compenser les variations de pH. Deux à quatre heures plus tard, le gel est délicatement décollé du bord de la boîte de culture à l'aide d'une aiguille à injection et transféré dans une boîte plus large, en présence d'un excès de milieu de culture pour une période de 12 à 24 heures. Pendant ce laps de temps, les fibroblastes inclus dans le gel de collagène s'attachent sur les fibres de collagène, ce qui induit une rétraction de la structure. Il est habituel de constater une réduction de plus de 50 % du volume initial. L'ensemble est alors prêt à être implanté dans l'organisme.

### 1.3. - Implantation

Le néo-organe est introduit dans la cavité péritonéale au cours d'une laparotomie effectuée sous anesthésie générale. Chez la souris, l'implantation est effectuée en insérant sans fixation le néo-organe entre les anses intestinales au contact du mésentère. Chez le gros mammifère, le néo-organe est fixé par deux points entre les deux feuillets du tablier épiploïque. Des connexions vasculaires s'établissent dès les premiers jours. La protéine exogène peut alors être libérée dans la circulation de l'animal receveur. A partir de la troisième semaine suivant l'implantation, un organe souvent pédiculé, encapsulé et bien individualisé est formé. De nombreuses connexions vasculaires sont visibles à sa surface. Il n'existe pas d'adhérence inflammatoire. Les examens après 3 et 6 mois montrent un aspect identique. L'analyse histologique à ce stade révèle la présence d'un tissu conjonctif lâche, vascularisé et peu inflammatoire.

### 1.4. - Traitement des maladies de surcharge lysosomale.

Ce procédé a été appliqué au traitement d'une maladie de surcharge lysosomale pour laquelle un modèle murin est disponible. Il s'agit d'un déficit en β-glucuronidase responsable d'une mucopolysaccharidose. La β-glucuronidase est phosphorylée et est donc, en partie, transportée vers les lysosomes et en partie, secrétée dans la circulation. Elle peut être captée par d'autres tissus, par l'intermédiaire du récepteur au mannose-6-phosphate exprimé à la surface de nombreuses cellules. Ce captage spécifique permet une approche thérapeutique où la distribution systémique continue de l'enzyme manquante est assurée par des cellules génétiquement modifiées.

Des implants de 0,5 cm³ contenant 20x10⁶ fibroblastes autologues sont bien tolérés par la souris. Les cellules implantées expriment le gène de la β-glucuronidase humaine inséré dans le vecteur M48, sous le contrôle du promoteur PGK et secrètent la protéine étrangère au-delà de 5 mois. L'effet biologique est objectivé par une chute rapide de l'élimination urinaire des catabolites intermédiaires de la dégradation des mucopolysaccharides. La normalisation histologique des tissus de l'animal receveur est spectaculaire, particulièrement au niveau du foie et de la rate. Elle est stable au cours du temps, et dépend de la présence du néo-organe dont l'exérèse induit un retour à l'état pathologique initial.

Ce procédé est applicable au traitement de l'ensemble des maladies de surcharge lysosomale humaines, y compris la maladie de Gaucher pour laquelle l'apport d'une forme soluble de la glucocérébrosidase est envisageable.

### Exemple 2 :

**Sécrétion d'une enzyme lysosomale (β-glucuronidase) chez le chien avec des implants contenant des fibres de PTFE, du collagène de queue de rat et des fibroblastes cutanés modifiés avec un vecteur rétroviral.**

Quatre chiens ont reçu de 1 à 6 néo-organes contenant chacun 10⁹ fibroblastes autologues génétiquement modifiés au moyen du vecteur M48-βglu, pour sécréter la β-glucuronidase humaine. Trois animaux de race Labrador pesant de 21 à 29 kg, et un animal de race Beagle pesant environ 9 kg ont été utilisés. L'implantation chirurgicale réalisée sous anesthésie générale a consisté à insérer les implants entre les deux feuillets pariétal et viscéral du tablier épiploïque à proximité de la grande courbure de l'estomac. Ce geste rapide s'acccompagne de suites opératoires simples, et permet d'envisager pour l'avenir une implantation par coeliochirurgie. Un mois et demi, quatre mois, et six mois après l'implantation, une laparotomie exploratrice a été réalisée afin de pratiquer un bilan macroscopique de l'implant. La mise à jour du tablier épiploïque a été facile et le repérage de la structure greffée s'est fait très rapidement. Aucune adhérence n'a été notée, et la vascularisation était importante, comportant des vaisseaux de gros calibre en connexion avec 1 'implant. L'aspect de l'implant ne s'est pas modifié pendant la durée de cette observation. Une biopsie hépatique pratiquée dans le même temps opératoire a permis de rechercher la présence de β-glucuronidase humaine. La mesure de l'activité sur des extraits cellulaires a montré un niveau équivalent à 2-3% de l'activité canine endogène, stable au cours du temps chez les animaux ayant reçu plus de 2 x 10⁹ fibroblastes autologues génétiquement modifiés. La révélation in situ sur coupe a montré une activité localisée dans les cellules de Küpffer. Les résultats sont résumés au tableau I.

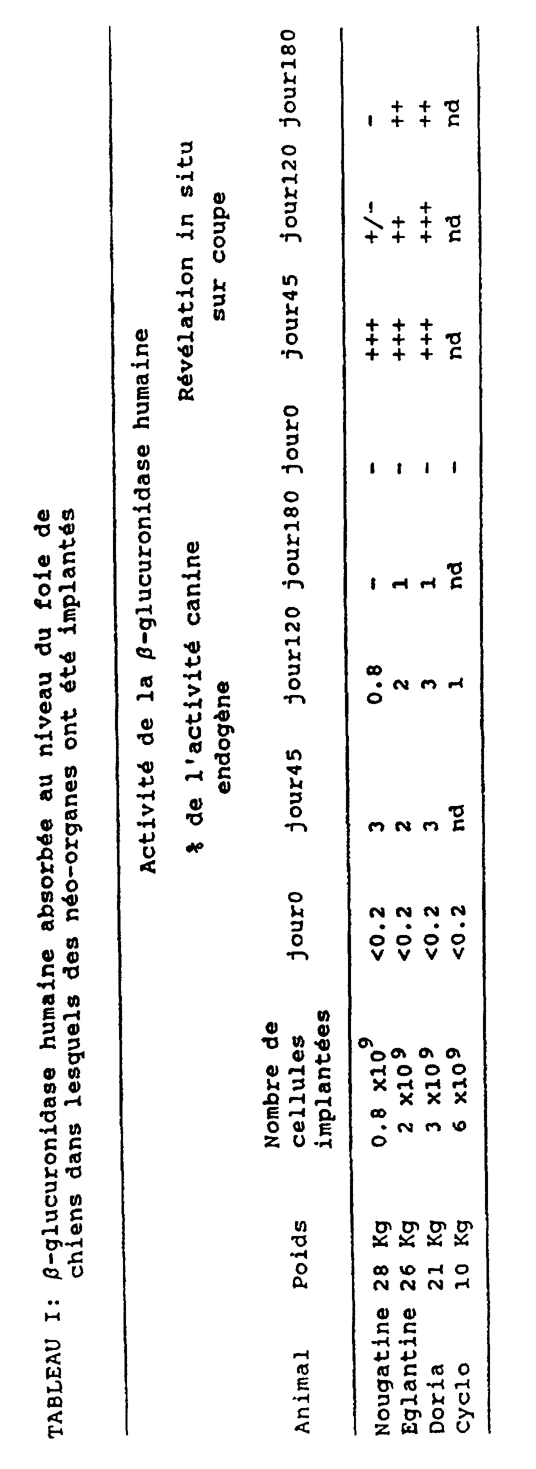

### Exemple 3 :

**Sécrétion d'une enzyme lysosomale (β-L-iduronidase) chez la souris avec des implants contenant des fibres de PTFE, du collagène de queue de rat et des fibroblastes cutanés modifiés avec un vecteur rétroviral.**

Un cDNA codant pour l'β-L-iduronidase humaine a été introduit dans le vecteur M48 et un rétrovirus recombinant a été produit dans la lignée ΨCRIP. Des fibroblastes de souris nude ont été placés en culture primaire et infectés par ce vecteur rétroviral. Les cellules secrétant l'enzyme humaine ont été introduites chez six receveurs syngéniques, dans deux néo-organes contenant chacun 10 millions de cellules.

Les animaux ont été sacrifiés après 35 à 77 jours et la présence d'enzyme humaine dans leur foie et leur rate a été vérifiée au moyen d'un anticorps monoclonal. Une activité enzymatique équivalente à 1-2% de l'activité endogène a été mise en évidence, indiquant la production de l'enzyme à partir des cellules du néo-organe et son captage à distance.

### Exemple 4 :

**Secrétion d'érythropoiétine (EPO) chez la souris avec des implants contenant des fibres de PTFE, du collagène de queue de rat et des fibroblastes cutanés modifiés avec un vecteur rétroviral;**

Le gène nlslacZ a été excise du vecteur M48-nlslacZ par digestion BamHI et remplacé par un cDNA codant pour l'EPO de souris synthétisé par PCR, réalisant ainsi le vecteur M48EPO. Un clone de cellule ΨCRE produisant des particules rétrovirales M48EPO a été isolé. Des fibroblastes ont été prélevés par biopsie cutanée chez des souris adultes et une culture primaire a été établie en milieu RPMI 1640 avec 10% de SVF. Ces cellules ont été infectées par le fecteur M48EPO de manière itérative durant les 4 premiers jours de la culture. L'analyse par Southern blot amontré que les cellules infectées contenaient en moyenne 2 copies du génome M48 EPO par cellule. L'analyse par Northern blot a montré une expression du cDNA de l'EPO très majoritairement à partir du promoteur PGK-1. Un essai biologique de l'activité EPO secrétée par ces cellules, ainsi qu'un test ELISA ont mesuré une secrétion de 17 unités d'EPO par million de cellules par 24 heures. Ces cellules ont été amplifiées en culture, trypsinées, puis resuspendues dans du RPMI 1640 à une concentration de 2,5 x 10⁷ à 2 x 10⁸ cellules par ml.

Des implants ont été préparés en associant les éléments suivants dans un puits de 0,9 cm²/ 1) des fibres de PTFE préalablement traitées comme décrit ci-dessus pour être recouvertes successivement de collagène de queue de rat (Sigma), d'héparine (Roche), et de bFGF (Promega) ; 2) 1 ml d'une solution contenant du collagène de queue de rat (Sigma) à x mg/ml, du bFGF (10µg/ml) dans du milieu RPMI 1640 ; 3) un volume de 10µl de la suspension de fibroblastes génétiquement modifiés pour secréter de l'EPO. Après avoir réalisé un mélange homogène, celui-ci est solidifié par incubation à 37°C pendant 30 minutes, puis le gel est délicatement séparé des parois du puits de culture, transféré dans une boîte de Petri de 35 mm de diamètre, recouvert de milieu RPMI 1640 contenant 10% de SVF, et incubé à 37°C pendant 3 jours. Une contraction du gel s'est opérée durant cette incubation résultant en une diminution de taille de 50%. Les implants ont ensuite été insérés dans la cavité péritonéale de 14 souris syngénéiques aux fibroblastes génétiquement modifiés.

La mesure hebdomadaire de l'hématocrite (valeur normale 46 ± 1,5%) a montré une élévation progressive vers un plateau dont le niveau variait en fonction du nombre de cellules secrétant de l'EPO dans les implants. Ce plateau était à 80% pour 2 x 10⁷ cellules, 70% pour 10⁷ cellules, 60% pour 5 x 10⁶ cellules, 52% pour 2,5 x 10⁶ cellules. Il s'est maintenu à ce niveau durant les 6 mois d'observation des animaux. Les concentrations d'EPO dans le sérum des souris porteuses d'implants secrétant de l'EPO étaient de 60 à 400 mU par ml (normale < 20 mU/ml).

Les implants constitués de PTFE, de collagène de queue de rat et de cellules génétiquement modifiées par le vecteur rétroviral M48EPO permettent donc une secrétion d'EPO in vivo stable à long terme, à des niveaux élevés estimés entre 500 et 1500 mU/kg/24 heures, c'est-à-dire suffisants chez l'homme pour la correction de l'anémie due aux hémoglobinopathies comme le drépanocytose et la β-thalassémie. DEs niveaux 10 fois moindres seraient suffisants pour le traitement de l'anémie associée à l'insuffisance rénale chronique.

### UTILISATION IN VIVO D'IMPLANTS COMPRENANT DU CORAIL A TITRE DE SUPPORT BIOLOGIQUE

### 1. Chez la souris

Chez la souris C3H/He des expériences de compatibilité du Biocoral® en tant qu'implant, ont montré qu'il pouvait représenter une alternative avantageuse par rapport à des supports synthétiques.

Le Biocoral® a pu en effet être efficacement recouvert de différents éléments de la matrice extra cellulaire ainsi que de facteurs angiogéniques. On a montré que le collagène de type I murin à une concentration de 0.5 mg/ml dans 0.001% d'acide acétique adhère et recouvre le support corallien après séchage à l'air ambiant pendant 12 à 24 hrs. La présence de cet élément de la matrice extra-cellulaire a ensuite permis la liaison irréversible de facteur basique de croissance des fibroblastes marqué avec ¹²⁵I ¹²⁵I-basic Fibroblast Growth Factor (bFGF) au Biocoral®. On a réalisé ainsi la fixation en moyenne de 50 ng de bFGF sur 40 mg de support corallien prétraité au collagène murin de type I. Bien que cette liaison ait pu s'effectuer aussi en l'absence de prétraitement au collagène, elle était dans ce cas réversible donc de faible affinité. Le collagène est donc un intermédiaire indispensable et justifié pour la fixation stable de facteurs angiogéniques au Biocoral. Enfin, la liaison du bFGF a pu être augmentée de cinq à dix fois lorsque le recouvrement de 40 mg de Biocoral par du collagène était complété par de l'héparan sulfate (Sigma) 200 µg (solution à 1 mg/ml dans du PBS) ou de l'heparine (Roche) 500 unités (solution à 5000 unités/ml). Ces deux composants de la matrice extra-cellulaire ont une affinité connue pour le collagène (Baird, A., Schubert, D., Ling, N., and Guillemin, R. (1988) Receptor and heparine binding domains of basic fibroblast growth factor. Proc. Natl. Acad. Sci. USA 85, 2324-2328).
Leurs caractères anionique et poly-sulfaté sont responsables de la forte affinité pour le bFGF (Johnson, D.E., Lee, P.L., Lu, J., and Williams, L.T. (1990) Diverse forms of a receptor for acidic and basic fibroblast growth factors. Mol. Cell. Biol. 10, 4728-4739). Après incubation en présence de l'un ou l'autre de ces composants, 1 hr à 4°C sous agitation, le Biocorail a été séché à l'air ambiant. Ainsi héparine, le corail a une action anticoagulante.

Des essais d'implantation sous-cutanée de corail sphérique de 40 mg, à haute porosité (50%), recouvert de collagène de type I murin et de bFGF (=50 ng/40 mg de corail) ont été réalisés in vivo sur une dizaine de souris. L'aspect macroscopique et histologique de ces implants a été analysé sur une période de 7 mois. Les résultats montrent que le Biocoral était progressivement résorbé en quelques mois et laissait place à une structure formée de tissu conjonctif vascularisé ayant la forme sphérique initiale de l'implant corallien avec cependant une réduction de volume d'approximativement un tiers. L'analyse histologique de ce tissu conjonctif à trois mois, montrait une vascularisation intense et l'absence totale de cellules inflammatoires en regard des résidus coralliens. Le tissu conjonctif proprement dit était formé de cellules mésenchymateuses peu denses, entourées de fibres de collagène néo-formé. L'analyse à 7 mois montrait des résultats semblables sans involution.

### 2. Chez le chien :

Chez un chien de 25 kg, nous avons réalisé une première expérience d'implantation de néo-organe dont la partie externe en corail poreux (50%) a permis de retenir un gel de collagène rétracté contenant 1 à 2 x 10⁹ fibroblastes autologues génétiquement modifiés. Le support en Biocoral avait été recouvert par du collagène murin de type I et de l'héparine (Roche). Après séchage, le corail ainsi prétraité a été incubé en présence de facteur de croissance angiogénique de type bFGF (Farmitalia) puis rincé en PBS. L'implantation chirurgicale réalisée sous anesthésie générale, a consisté à insérer l'ensemble entre les deux feuillets pariétal et viscéral du tablier épiploïque à proximité de la grande courbure de l'estomac. Ce geste rapide a permis des suites opératoires simples, et laisse envisager pour l'avenir une implantation par coeliochirurgie. Quarante cinq jours après, une laparotomie exploratrice a été réalisée afin de pratiquer un bilan macroscopique de l'implant. La mise à jour du tablier épiploïque a été facile, sans adhérence et le repérage de l'implant corallien, contenant les fibroblastes autologues génétiquement modifiés, s'est fait très rapidement. Aucune adhérence n'a pu être notée, et la vascularisation était importante, comportant des vaisseaux de gros calibre en connexion avec l'implant. Un processus de résorption progressive du corail a été observé pendant 4 mois au moins.

### 3. Autres mammifères :

La création de néo-organes, en utilisant du Biocoral, adaptés à l'implantation chirurgicale sur de gros mammifères, notamment chez l'homme, en vue d'un développement clinique est réalisée de façon analogue. Le Biocoral est modulable dans sa conception, capable de déclencher une angiogénèse soutenue associée à sa propre résorption et enfin dénué d'effets systémiques et locaux de type inflammatoire. Un tel bio-matériau sert avantageusement de receptacle et de squelette à un gel de collagène de type I contenant des cellules autologues génétiquement modifiées, capables de sécréter un facteur thérapeutique dans la circulation une fois les connexions vasculaires établies.

### Exemple 5 :

**Secrétion d'érythropoiétine (EPO) chez la souris avec des implants contenant des fragments de corail, du collagène de queue de rat et des fibroblastes cutanés modifiés avec un vecteur rétroviral.**

De la même façon que dans l'exemple 4, des fibroblastes de souris ont été obtenus à partir d'une biopsie cutanée, mis en culture, et transduits avec le vecteur M48EPO. Après amplification, les cellules ont été trypsinées et resuspendues dans du milieu RPMI 1640 avec 10% de SVFR à la concentration de 10⁸ cellules par ml.

Des implants ont été préparés en associant les éléments suivants dans un puits de 0,9 cm² : 1) de la poudre de corail (Inoteb) formée de particules de porosité ≥ 45% et de granulométrie entre 600 et 1000 µm dont les pores avaient un diamètre moyen de 150 µm préalablement traitées par une incubation dans une solution de collagène de queue de rat (1 mg/ml) pendant 30 minutes à température ambiante, séchées, puis incubées dans une solution d'héparine (Roche) pendant 30 minutes à température ambiante, séchées, puis incubées dans une solution de bFGF (Promega) à 100 µg/ml pendant 30 minutes à température ambiante ; 2) 1 ml d'une solution contenant du collagène de queue de rat (Sigma) à 3 mg/ml, du bFGF (100 µg/ml) dans du milieu RPMI 1640 ; 3) un volume de 100 µl de la suspension de fibroblastes génétiquement modifiés pour secréter de l'EPO. Comme dans les exemples 1 à 3, un mélange homogène de ces constituants a été solidifé par incubation à 37°C pendant 30 minutes, puis le gel a été délicatement séparé des parois du puits de culture, transféré dans une boîte de Petri de 35 mm de diamètre, recouvert de milieu RPMI 1640 contenant 10% de SVF, et incubé à 37°C pendant 3 jours. Les implants ont ensuite été insérés dans la cavité péritonéale de 3 souris syngénéiques aux fibroblastes génétiquement modifiés.

Comme dans l'exemple 4, la mesure de l'hématocrite des animaux receveurs a montré une augmentation rapide atteignant un plateau après 3 semaines, et se maintenant à ce niveau pendant toute la durée de l'observation (2 mois). Des implants formés d'un gel de collagène structuré avec des fragments de corail permettent donc le maintien à l'état fonctionnel de fibroblastes génétiquement modifiés implantés dans la cavité péritonéale d'animaux receveurs, ainsi que la secrétion à long terme d'une protéine soluble dans le sérum.

### Exemple 6 :

**Sécrétion d'érythropoiétine (EPO) chez la souris avec des implants contenant des fibres de collagène réticulé, du collagène de queue de rat et des fibroblastes cutanés modifiés avec un vecteur rétroviral.**

Une expérience semblable aux exemples 4 et 5 a consisté à réaliser des implants associant des fibroblastes de souris transduits avec le vecteur M48EPO, du collagène de queue de rat, et un support formé de fibres de collagène réticulé (Imedex). La préparation a été réalisée de la façon suivante : 1) les fibres déshydratées et stérilisées par rayonnement ont d'abord été réhydratées par une incubation de 12 heures à +4°C dans du milieu RPMI 1640. Elles ont ensuite été incubées à température ambiante pendant 30 minutes dans du milieu RPMI contenant 10% de SVF et 100 µg/ml de bFGF (Promega), puis disposées dans un puits de 0,4 cm² dans lequel a été ajouté un ml d'une solution de RPMI 1640 contenant du SVF (10%) , du collagène de queue de rat (1 mg/ml), du bFGF (100 µg/ml, Promega), et 10⁷ fibroblastes génétiquement modifiés pour secréter de l'EPO. Après solidification à 37°C pendant 30 minutes, le gel a été délicatement décollé des parois du puits de culture, transféré dans une boîte de Petri de 35 mm de diamètre, et recouvert de milieu RPMI 1640 avec 10% de SVF. Les implants ont été incubés 3 jours à 37°C, une contraction modérée de la structure a été observée, puis ils ont été implantés chez 3 souris syngénéiques.

De manière similaire à ce qui a été décrit dans les exemples 4 et 5, la mesure hebdomadaire de l'hématrocrite a montré une élévation progressive jusqu'à un niveau plateau qui s'est maintenu pendant la durée de l'observation (2 mois). Des implants formés d'un gel de collagène structuré avec des fibres de collagène réticulé permettent donc le maintien à l'état fonctionnel de fibroblastes génétiquement modifiés implantés dans la cavité péritonéale d'animaux receveurs, ainsi que la secrétion à long terme d'une protéine soluble dans le sérum.

### Exemple 7 :

**Détoxification de la bilirubine par glucurono-conjugaison chez des rats porteurs d'implants contenant des fibres de PTFE, du collagène de queue de rat, et des fibroblastes génétiquement modifiés exprimant l'enzyme UDP-glucuronosyl transférase.**

La bilirubine est un produit de dégradation de l'hème produit de manière permanente et en quantité importante par l'organisme. La bilirubine est liposoluble, et peut pénétrer passivement dans les cellules, où son accumulation est toxique. L'élimination de la bilirubine est réalisée par glucurono-conjugaison au niveau des hépatocytes par l'enzyme bilirubine UDP-glucuronosyl transférase (bil. UDPGT) dont il existe deux isoformes connues (1 et 2). L'addition d'acide glucuronique rend la molécule hydrosoluble, non toxique, et permet son excrétion dans la bile. Le déficit héréditaire total de l'activité bil.UDPGT est responsable d'une maladie gravissime (maladie de Crigler-Najjar) qui est redevable d'une transplantation hépatique. Il existe un modèle de cette maladie chez le rat (rat Gunn).

Le cDNA de rat codant pour l'isoforme 2 (bil.UDPGT-2) et le cDNA humain codant pour l'isoforme 1 (bil.UDPGT-1) ont été insérés dans le vecteur M48 après excision du gène nlslacZ par l'enzyme BamHI, générant ainsi les vecteurs M48GT-2 et M48GT-1. De manière semblable à ce qui a été décrit dans les exemples 1, 3 et 4 des fibroblastes de rat Gunn ont été prélevés par une biopsie cutanée, mis en culture, et transduits par le vecteur M48GT-2. La présence d'en moyenne une copie du génome vecteur dans les cellules transduites a été vérifiée par Southern blot, et l'expression à partir du promoteur PGK a été vérifiée par Northern Blot. L'activité enzymatique bil.UDPGT a été détectée dans les extraits microsomaux des fibroblastes infectés. Les cellules ont été amplifiées en culture, trypsinées, puis resuspendues dans du RPMI 1640 à une concentration de 2 x 10⁸ cellules par ml.

Des implants contenant 2 x 10⁷ cellules génétiquement modifiées ont été préparés de la même façon que dans les exemples 1, 3 et 4. Deux implants ont été insérés dans la cavité péritonéale de chaque rat Gunn receveur. Comme la colonie de rats Gunn utilisée n'est pas consanguine, il a été pris soin que chaque animal soit réimplanté avec ses propres cellules. La mesure des concentrations de bilirubine non conjuguée chez les animaux traités et chez des rats témoins du même âge a fait apparaître une diminution significative (entre 20 et 50%) et stable pendant une observation de 2 mois chez les rats porteurs d'implants de fibroblastes exprimant la bil.UDPGT. Des échantillons de bile ont été recueillis au moment du sacrifice des animaux et analysés par chromatographie liquide à haute pression (HPLC). Alors que les animaux non traités montrent une totale absence de bilirubine conjuguée dans la bile, les rats Gunn porteurs d'implants contenant des fibroblastes exprimant la bil.UDPGT présentaient des pics identifiés comme de la bilirubine mono et diconjuguée. La proportion de bilirubine conjuguée atteignait jusqu'à 11% de la bilirubine présente dans la bile.

Ces résultats indiquent que des fibroblastes de rat transduits avec le vecteur M48UDPGT-1 et greffés chez des rats Gunn sous la forme d'implants associant des fibres de PTFE et du collagène de queue de rat, sont capables de réaliser in vivo la réaction de glucurono-conjugaison de la bilirubine, laquelle bilirubine conjuguée peut ensuite être éliminée dans la bile par les hépatocytes, permettant ainsi une correction partielle du phénotype des animaux malades. Il est donc possible de réaliser la détoxification de produits biologiques in vivo grâce à des fibroblastes génétiquement modifiés implantés dans un gel de collagène structuré avec des fibres de PTFE.

### Exemple 8 :

**Utilisation d'un promoteur inductible (Mx) pour moduler l'expression d'un cDNA inséré dans un vecteur rétroviral.**

Dans le cas d'implants secrétant de manière continue une protéine dans la circulation, il serait souhaitable de pouvoir exercer un contrôle sur cette sécrétion. Pour ce faire un vecteur rétroviral dérivé du vecteur M48 a été construit dans lequel le promoteur PGK-1 a été excisé et remplacé par un fragment de 250 bp couvrant la région -250 / +1 du promoteur du gène Mx de la souris. Ce promoteur comprend des éléments de réponse à la stimulation par les interférons α et β. Chez la souris la protéine Mx est exprimée en réponse à une secrétion d'interféron α ou β. De la même façon que dans les exemples 3, 4 et 5, le cDNA codant pour l'EPO de souris a été inséré dans ce vecteur au site BamHI, générant ainsi le vecteur MxEPO. Des cellules ΨCRE produisant des particules rétrovirables écotropes recombinantes contenant le génome du vecteur MxEPO ont été isolées. Des fibroblastes isolés d'une biopsie cutanée réalisée chez une souris ont été infectés avec le vecteur MxEPO, et l'EPO sécrétée dans le surnageant de ces cultures a été mesurée avant et après addition d'interféron α (1000 unités par ml) dans le milieu de culture. Les niveaux de base de secrétion d'EPO étaient inférieurs à 0,3 unités/ml/24 heures témoignant d'une faible activité du promoteur Mx présent dans le vecteur MxEPO en l'absence d'interféron. Une augmentation de la secrétion jusqu'à 10 fois le niveau de base a été mesurée après addition d'interféron α. Cette secrétion élevée s'est maintenue pendant au moins 6 jours. Cette observation montre qu'un contrôle peut être exercé sur un promoteur transcriptionnel inséré dans un vecteur rétroviral, après l'intégration de celui-ci dans le génome de la cellule cible.

Des implants constitués de fibres de PTFE, de collagène de queue de rat, et contenant des fibroblastes cutanés transduits avec le vecteur MxEPO ont été préparés et implantés chez des souris syngénéiques. En l'absence d'autre traitement, ces animaux ont maintenu un hématocrite normal. Certains des animaux receveurs ont été traités par l'injection intra-péritonéale de 50 mg de polyA-U (Boehringer) chaque 48 heures pendant deux semaines afin de provoquer une élévation de l'hématocrite.

### Traitement de maladies génétiques ou acquises.

Le procédé est applicable à la distribution dans le sérum de toute protéine soluble, ou de protéines transmembranaires tronquées de leur domaine d'immobilisation dans la membrane. Cependant, il ne permet pas une régulation des concentrations sériques de la protéine étrangère. Les applications suivantes sont envisagées pour les maladies génétiques : dans l'hémophilie B, l'apport de facteur IX ; dans l'hémophilie A, l'apport de facteur VIII sous forme d'une protéine délétée dans la région B ; dans la β-thalassémie, l'apport d'érythropoïétine susceptible de corriger l'anémie par stimulation de la synthèse d'hémoglobine foetale.

L'apport de CD4 soluble, ou de ses dérivés couplés à des immunotoxines ou des immunoglobulines, est envisageable dans les infections par des rétrovirus de la famille du VIH. Plus généralement, toute protéine anti-virale soluble pourrait être distribuée de cette façon.

### Production d'anticorps

La distribution de protéine étrangère chez la souris induit une réponse immunitaire marquée par l'apparition d'anticorps spécifiques, comme nous l'avons observé pour la β-glucuronidase, le CD4 soluble, et l'érythropoïétine. Le procédé pourrait donc être appliqué pour immuniser des animaux contre le produit non encore identifié d'un cDNA isolé par génétique inverse ou par d'autres moyens. Des anticorps polyclonaux et monoclonaux pourraient être obtenus de cette manière, sans nécessiter la préparation préalable de la protéine.

La même approche est envisageable pour réaliser des immunisations dans un but thérapeutique ou préventif. Des applications vaccinales pourraient être proposées, par exemple, contre des épitopes neutralisants de l'enveloppe du VIH ou des virus apparentés.

### Régulation par le système tel opérateur/tet répresseur

On prépare des fibroblastes cutanés dans lesquels on introduit deux vecteurs rétroviraux, l'un codant pour la protéine d'intérêt placé sous le contrôle d'un promoteur comportant les éléments tet opérateur, l'autre codant pour le tet répresseur ou une molécule apparentée modifiée pour produire un effet activateur. Suivant la nature de ce dernier élément et celle du promoteur contenant les séquences opératrices, l'addition de tétracycline induira une activation ou une répression de la transcription. Les fibroblastes transduits avec ces deux vecteurs sont implantés in vivo dans un néo-organe, et les animaux sont traités par de la tétracycline pour évaluer l'induction de l'expression. Le gène rapporteur est de préférence celui de l'EPO de souris.

### Régulation par le récepteur de la progestérone modifé pour induire la transcription en présence de RU486.

Le récepteur de la progestérone lorsqu'il est tronqué de sa région C-terminale devient capable de stimuler la transcription en présence de RU486. Des fibroblastes cutanés sont infectés avec deux vecteur rétroviraux, l'un codant pour la protéine d'intérêt exprimé sous contrôle d'un promoteur comportant des éléments de réponse à la progestérone, l'autre codant pour une version tronquée du récepteur de la progestérone. Comme précédemment, les fibroblastes transduits avec ces deux vecteurs sont implantés dans des néo-organes chez la souris, et les animaux sont traités par le RU486.

## Revendications

1. Implant **caractérisé en ce qu'**il comprend :
- un support biocompatible à base de calcaire permettant l'ancrage biologique de cellules,
- des cellules ayant la capacité d'exprimer et de sécréter naturellement ou après recombinaison un composé déterminé, par exemple un composé ayant un intérêt thérapeutique ; et
- un constituant capable d'induire et/ou de favoriser la gélification desdites cellules.

2. Implant selon la revendication 1, **caractérisé en ce que** le support biocompatible est un support de type résorbable in vivo, au moins partiellement.

3. Implant selon l'une quelconque des revendications 1 à 2 **caractérisé en ce que** le support biocompatible est un support à base de carbonate de calcium, de préférence **en ce qu'**il s'agit de corail.

4. Implant selon la revendication 3, **caractérisé en ce que** le support biocompatible est du corail à haute porosité.

5. Implant selon la revendication 4, **caractérisé en ce que** le corail à haute porosité est du corail sphérique.

6. Implant selon l'une quelconque des revendications 1 à 5, **caractérisé en ce que** le constituant capable d'induire et/ou de favoriser la gélification des cellules est du collagène, en particulier du collagène de type I, de préférence à une concentration de l'ordre de 1,5 mg/ml.

7. Implant selon l'une quelconque des revendications 1 à 6, **caractérisé en ce que** le constituant capable d'induire et/ou de favoriser la gélification des cellules est choisi parmi des supports à base de :
- collagène non réticulé,
- alginates.

8. Implant **caractérisé en ce qu'**il comprend :
- un support biocompatible à base de corail permettant l'ancrage biologique de cellules,
- des cellules recombinantes ayant la capacité d'exprimer et de sécréter un composé déterminé, et,
- du collagène de type I.

9. Implant selon l'une quelconque des revendications 1 à 8, **caractérisé en ce que** les cellules sont des cellules recombinantes ayant la capacité d'être tolérées immunologiquement par un organisme auquel elles seraient administrées, modifiées par une séquence de nucléotides codant pour un polypeptide déterminé.

10. Implant selon la revendication 9, **caractérisé en ce que** les cellules recombinantes sont des fibroblastes, en particulier des fibroblastes cutanés.

11. Implant selon l'une quelconque des revendications 1 à 10, **caractérisé en ce que** lesdites cellules sont des cellules recombinantes modifiées par un vecteur rétroviral comprenant une séquence d'ADN de provirus modifiée de façon telle que :
- les gènes gag, pol et env de l'ADN du provirus ont été supprimés au moins en partie et ce afin d'obtenir un ADN proviral incapable de se répliquer, cet ADN ne pouvant, en outre, être recombiné pour former un virus sauvage,
- la séquence LTR comporte une délétion dans la séquence U3, telle que la transcription sous forme d'ARNm qu'elle contrôle est réduite de manière significative, par exemple d'au moins 10 fois, et le vecteur rétroviral comprend, en outre, une séquence de nucléotides exogène sous le contrôle d'un promoteur, par exemple d'un promoteur constitutif ou inductible, exogène.

12. Implant selon la revendication 11, **caractérisé en ce que** l'ADN proviral du vecteur est issu du rétrovirus MuLV.

13. Implant selon la revendication 10 ou la revendication 11, **caractérisé en ce que** les séquences des gènes pol et env de l'ADN proviral sont entièrement supprimées.

14. Implant selon l'une quelconque des revendications 10 à 12, **caractérisé en ce que** la région U3 du fragment LTR3' de l'ADN proviral est supprimée au niveau du nucléotide 2797 de la figure 1.

15. Implant selon l'une quelconque des revendications 11 à 14, **caractérisé en ce que** la séquence de nucléotides exogène est sous le contrôle du promoteur PGK-1 de souris ou du promoteur PGK-1 humain, le cas échéant dépourvu de "TATA box".

16. Implant selon l'une quelconque des revendications 11 à 15, **caractérisé en ce que** la séquence provirale en amont du promoteur exogène est la séquence de nucléotides du provirus, située entre les nucléotides 1 et 1500 environ de la séquence représentée à la figure 1.

17. Implant selon l'une quelconque des revendications 11 à 16, **caractérisé en ce que** le vecteur rétroviral est le vecteur pM48 représenté à la figure 2, modifié par l'insertion de la séquence de nucléotides exogène au site BamHI.

18. Implant selon l'une quelconque des revendications 1 à 9 ou 11 à 17, **caractérisé en ce que** les cellules recombinantes sont des cellules tumorales.

19. Implant selon l'une quelconque des revendications 1 à 18, **caractérisé en ce que** les cellules recombinantes sont modifiées par un vecteur contenant une séquence ou plusieurs séquences de nucléotides exogènes, codant pour un antigène ou un déterminant antigénique ou codant pour un polypeptide ou une glycoprotéine, soluble dans le sérum, par exemple pour un polypeptide ou une glycoprotéine d'intérêt thérapeutique, notamment pour une hormone, une protéine ou une glycoprotéine de structure ou une protéine ou une glycoprotéine du métabolisme ou une protéine ou une glycoprotéine virale ou une protéine ayant les caractéristiques d'un anticorps ou d'un fragment d'anticorps.

20. Implant selon l'une quelconque des revendications 1 à 19, **caractérisé en ce qu'**il contient en outre un ou plusieurs facteurs angiogéniques, en particulier du bFGF.

21. Implant selon l'une quelconque des revendications 1 à 20, **caractérisé en ce qu'**il contient de l'héparine ou un dérivé héparinique.

22. Implant selon l'une quelconque des revendications 1 à 21, **caractérisé en ce qu'**il comprend de 10⁶ à 10⁹, de préférence de 5 x 10⁶ à 10⁷ cellules recombinantes.

23. Utilisation d'un implant selon l'une quelconque des revendications 1 à 22, pour la préparation d'une composition destinée à l'implantation de façon permanente ou transitoire, chez l'homme ou l'animal.

24. Composition **caractérisée en ce qu'**elle comprend un implant selon l'une quelconque des revendications 1 à 22, avec une ou plusieurs substances, notamment un antigène ou un adjuvant.

25. Utilisation d'un implant selon l'une quelconque des revendications 1 à 22 pour la préparation d'un composé actif pour le traitement de maladies génétiques, de maladies acquises ou de tumeurs.

26. Utilisation d'un implant selon l'une des revendications 1 à 22 pour la préparation d'une composition destinée à la sécrétion d'érythropoïétine.

27. Méthode de préparation d'un implant selon l'une quelconque des revendications 1 à 22, ladite méthode comprenant les étapes de :
- mise en contact du support biocompatible à base de calcaire avec lesdites cellules et un constituant capable d'induire et/ou de favoriser leur gélification ;
- incubation de la préparation obtenue à l'étape précédente afin d'obtenir la gélification desdits constituants ;
- culture des cellules ainsi obtenues dans des conditions leur permettant de s'attacher aux constituants gélifiés, et
- récupération de l'implant ainsi obtenu.

28. Méthode selon la revendication 27, **caractérisée en ce que** le support biocompatible est mis en contact avec des cellules préalablement incorporées à une solution de collagène.

29. Méthode selon la revendication 27 ou 28, **caractérisée en ce que** le support biocompatible est constitué de poudre de corail, préalablement traitée avec une solution de collagène et un facteur de croissance.

30. Kit pour la préparation d'un implant pour réaliser in vivo l'expression et la sécrétion par des cellules d'un composé pour produire un effet thérapeutique recherché, ledit kit comprenant :
- un support biocompatible à base de calcaire permettant l'ancrage biologique desdites cellules ; et
- un constituant capable d'induire et/ou de favoriser la gélification desdites cellules.

31. Kit selon la revendication 30, **caractérisé en ce que** le support biocompatible est un support à base de carbonate de calcium, de préférence de corail.

32. Kit selon la revendication 30 ou 31, **caractérisé en ce que** le constituant capable d'induire et/ou de favoriser la gélification des cellules est du collagène, en particulier du collagène de type I, de préférence à une concentration de l'ordre de 1,5 mg/ml.

33. Kit selon la revendication 30, **caractérisé en ce qu'**il comprend un ADN comprenant une séquence codant pour le composé exprimé et sécrété par lesdites cellules.

34. Kit selon l'une quelconque des revendications 30 à 33, **caractérisé en ce qu'**il comprend des cellules ayant la capacité d'exprimer et de sécréter naturellement ou après recombinaison un composé déterminé, par exemple un composé ayant un intérêt thérapeutique.

35. Kit selon la revendication 34, **caractérisé en ce que** les cellules sont des cellules recombinantes ayant la capacité d'être tolérées immunologiquement par un organisme auquel elles seraient administrées, modifiées par un vecteur rétroviral comprenant :
. une séquence d'ADN de provirus modifiée de façon telle que :
- les gènes gag, pol et env de l'ADN du provirus ont été délétés au moins en partie et ce afin d'obtenir un ADN proviral incapable de se répliquer, cet ADN ne pouvant, en outre, être recombiné pour former un virus sauvage,
la séquence LTR comporte une délétion dans la séquence U3, telle que la transcription sous forme d'ARNm qu'elle contrôle est réduite de manière significative, par exemple d'au moins 10 fois, et
. le vecteur rétroviral recombinant comprenant, en outre, une séquence de nucléotides exogène sous le contrôle d'un promoteur, par exemple d'un promoteur constitutif ou inductible, exogène.

36. Kit selon la revendication 35, **caractérisé en ce que** les cellules recombinantes sont des fibroblastes recombinants, notamment des fibroblastes cutanés.

37. Kit selon la revendication 35, **caractérisé en ce que** les cellules recombinantes sont des cellules tumorales.

38. Kit selon l'une quelconque des revendications 35 à 37, **caractérisé en ce que** les cellules recombinantes contiennent une séquence de nucléotides exogène qui code pour une protéine dont on souhaite obtenir l'expression, notamment pour une protéine soluble dans le sérum.

39. Kit selon l'une quelconque des revendications 35 à 37, **caractérisé en ce que** les cellules recombinantes contiennent une séquence de nucléotides exogène qui code pour la β-glucuronidase.

40. Kit selon la revendication 31, **caractérisé en ce qu'**il comprend un vecteur rétroviral recombinant comprenant :
. une séquence d'ADN de provirus modifiée de façon telle que :
- les gènes gag, pol et env de l'ADN du provirus ont été délétés au moins en partie et ce afin d'obtenir un ADN proviral incapable de se répliquer, cet ADN ne pouvant, en outre, être recombiné pour former un virus sauvage,
- la séquence LTR comporte une délétion dans la séquence U3, telle que la transcription sous forme d'ARNm qu'elle contrôle est réduite de manière significative, par exemple d'au moins 10 fois, et
. le vecteur rétroviral recombinant comprenant, en outre, une séquence de nucléotides exogène sous le contrôle d'un promoteur, par exemple d'un promoteur constitutif ou inductible, exogène.

41. Kit selon la revendication 40, **caractérisé en ce que** la séquence d'ADN de provirus modifiée, la séquence de nucléotides exogène et le promoteur exogène sont portés par un plasmide.

42. Kit selon la revendication 40, **caractérisé en ce que** l'ADN proviral est issu du rétrovirus MuLV.

43. Kit selon la revendication 40, **caractérisé en ce que** les séquences des gènes pol et env de l'ADN proviral sont entièrement supprimées.

44. Kit selon l'une quelconque des revendications 40 à 43, **caractérisé en ce que** dans le vecteur rétroviral, la région U3 du fragment LTR3' est supprimée au niveau du nucléotide 2797 de la figure 1.

45. Kit selon l'une quelconque des revendications 40 à 43, **caractérisé en ce que** dans le vecteur rétroviral, la séquence de nucléotides exogène est sous le contrôle du promoteur PGK-1 de souris ou du promoteur PGK-1 humain, le cas échéant dépourvu de "TATA box".

46. Kit selon l'une quelconque des revendications 40 à 44, **caractérisé en ce que** dans le vecteur rétroviral, la séquence provirale en amont du promoteur exogène est la séquence de nucléotides du provirus, située entre les nucléotides 1 et 1500 environ de la séquence représentée à la figure 1.

47. Kit selon la revendication 46, **caractérisé en ce que** dans le vecteur rétroviral, la séquence de nucléotides exogène est insérée au site BamHI en aval du promoteur PGK-1 constitutif exogène.

48. Kit selon l'une quelconque des revendications 40 à 46 **caractérisé en ce que** le vecteur rétroviral est le vecteur pM48 représenté à la figure 2, modifié par l'insertion de la séquence de nucléotides exogène au site BamHI.

49. Kit selon l'une quelconque des revendications 39 à 45, **caractérisé en ce que** le vecteur rétroviral contient au site BamHI en aval du promoteur constitutif exogène, un fragment BamHI du gène de la β-galactosidase.

50. Kit selon l'une quelconque des revendications 40 à 49, **caractérisé en ce que** le vecteur rétroviral contient en outre, en amont du promoteur constitutif exogène, une séquence d'activateur.

## Claims

1. Implant **characterized in that** it comprises:
- a calcium-based biocompatible support permitting the biological anchoring of cells,
- cells having the capacity to express and secrete naturally or after recombination a defined substance, for example a substance having therapeutic value, and
- a constituent capable of inducing and/or promoting the gelation of said cells.

2. Implant according to Claim 1, **characterized in that** the biocompatible support is a support of the type resorbable in vivo at least partially.

3. Implant according to anyone of Claims 1 to 2, **characterized in that** the biocompatible support is based on calcium carbonate, preferably it is coral.

4. Implant according to Claim 3, **characterized in that** the biocompatible support is high-porosity coral.

5. Implant according to Claim 4, **characterized in that** the high-porosity coral is a spherical coral.

6. Implant according to any one of the Claims 1 to 5, **characterized in that** the constituent capable of inducing and/or promoting the gelation of the cells is collagen, in particular type I collagen, preferably at a concentration of the order of 1.5mg/ml.

7. Implant according to any one of the Claims 1 to 6 **characterized in that** the constituent capable of inducing and/or promoting the gelation of the cells is selected from supports based on:
- uncross-linked collagen,
- alginates.

8. An implant **characterized in that** it comprises:
- a biocompatible support based on coral permitting the biological anchoring of cells;
- recombinant cells having the capacity to express and secrete a defined substance, and,
- type I collagen.

9. Implant according to any one of the Claims 1 to 8, **characterized in that** the cells are recombinant cells having the capacity to be tolerated immunologically by an organism to which they are administered, modified by a nucleotide sequence coding for a defined polypeptide.

10. Implant according to Claim 9, **characterized in that** the recombinant cells are fibroblasts, in particular skin fibroblasts.

11. Implant according to any one of the Claims 1 to 10, **characterized in that** said cells are recombinant cells modified by a retroviral vector comprising a proviral DNA sequence modified in a manner such that:
- the gag, pol and env genes of the proviral DNA have been deleted at least in part in order to produce a proviral DNA incapable of replicating, this DNA being in addition incapable of recombining to form a wild-type virus,
- the LTR sequence contains a deletion in the sequence U3 such that transcription of the mRNA that it controls is reduced significantly for example at least 10-fold, and the recombinant retroviral vector comprises, in addition, an exogenous nucleotide sequence under the control of a promoter for example an exogenous, constitutive or inducible promoter.

12. Implant according to Claim 11, **characterized in that** the proviral DNA of the vector is derived from the MuLV retrovirus.

13. Implant according to Claim 10 or Claim 11, **characterized in that** the sequences of the pol and env genes of the proviral DNA are entirely deleted.

14. Implant according to any one of the Claims 10 to 12, **characterized in that** the U3 region of the LTR3' fragment of the proviral DNA is deleted at the level of nucleotide 2797 of Figure 1.

15. Implant according to any one of the Claims 11 to 14, **characterized in that** the exogenous nucleotide sequence is under the control of the mouse PGK-1 promoter or the human PGK-1 promoter, optionally lacking a "TATA box".

16. Implant according to any one of the Claims 11 to 15, **characterized in that** the proviral sequence upstream from the exogenous promoter is the proviral nucleotide sequence situated between nucleotides 1 and about 1500 of the sequence shown in Figure 1.

17. Implant according to any one of the Claims 11 to 16, **characterized in that** the retroviral vector is the vector pM48 shown in Figure 2, modified by the insertion of the exogenous nucleotide sequence at the BamHI site.

18. Implant according to any one of the Claims 1 to 9, or 11 to 17, **characterized in that** the recombinant cells are tumor cells.

19. Implant according to any one of the Claims 1 to 18, **characterized in that** the recombinant cells are modified by a vector containing one or more exogenous nucleotide sequences coding for an antigen or an antigenic determinant soluble in the serum, for example for a polypeptide or glycoprotein of therapeutic interest, in particular a hormone, a structural protein or glycoprotein or a protein or glycoprotein or a viral protein or glycoprotein or a protein having the characteristics of an antibody or an antibody fragment.

20. Implant according to any one of the Claims 1 to 19, **characterized in that** it contains in addition one or more angiogenic factors, in particular bFGF.

21. Implant according to any one of the Claims 1 to 20, **characterized in that** it contains heparin or a heparin derivative.

22. Implant according to any one of the Claims 1 to 21, **characterized in that** it comprises from 106 to 109, and preferably from 5 x 10⁶ to 10⁷ recombinant cells.

23. Use of an implant according to any one of the Claims 1 to 22, in the preparation of a composition intended for the implantation, in a permanent or temporary fashion in man or animals.

24. Composition **characterized in that** it contains an implant according to any one of the Claims 1 to 22 with one or more substances, in particular an antigen or an adjuvant.

25. Use of an implant according to any one of Claims 1 to 22 in the preparation of an active substance for the treatment of genetic diseases, acquired diseases or tumors.

26. Use of an implant according to any one of Claims 1 to 22 for the preparation of a composition intended for erythropoïetin secretion.

27. Method of preparation of an implant according to any one of the Claims 1 to 22, said method comprising the steps of:
- placing of the calcium-based biocompatible support in contact with said cells and a constituent capable of inducing and/or promoting their gelation;
- incubation of the preparation obtained in the previous step in order to obtain the gelation of said constituents;
- culture of the cells thus obtained under conditions allowing them to bind to the gelled constituents, and
- recovery of the implant thus obtained.

28. Method according to Claim 27, **characterized in that** the biocompatible support is placed in contact with cells previously incorporated into a solution of collagen.

29. Method according to Claim 27 or 28, **characterized in that** the biocompatible support is constituted of coral powder, previously treated with a solution of collagen and a growth factor.

30. Kit for the preparation of an implant to achieve the in vivo expression and secretion by cells of a substance to produce a desired therapeutic effect, said kit containing:
- a calcium-based biocompatible support making possible the biological anchoring of said cells; and
- a constituent capable of inducing and/or promoting the gelation of said cells.

31. Kit according to Claim 30, **characterized in that** the biocompatible support is a calcium carbonate-based support, preferably coral.

32. Kit according to Claim 30 or 31, **characterized in that** the constituent capable of inducing and/or promoting the gelation of the cells is collagen, in particular type I collagen, preferably at a concentration of the order of 1.5 mg/ml.

33. Kit according to Claim 30, **characterized in that** it contains a DNA comprising a sequence coding for the substance expressed and secreted by said cells.

34. Kit according to any of Claims 30 to 33, **characterized in that** it comprises cells having the capacity to express and secrete naturally or after recombination a defined substance for example a substance having a therapeutic value.

35. Kit according to Claim 34, **characterized in that** said cells are recombinant cells having the capacity to be tolerated immunologically by an organism in which they might be implanted, modified by a retroviral vector comprising a proviral DNA sequence modified in a manner such that:
- the gag, pol and env genes of the proviral DNA have been deleted at least in part in order to produce a proviral DNA incapable of replicating, this DNA being in addition incapable of recombining to form a wild-type virus,
- the LTR sequence contains a deletion in the sequence U3 such that transcription of the mRNA that it controls is reduced significantly for example at least 10-fold, and the recombinant retroviral vector comprises, in addition, an exogenous nucleotide sequence under the control of a promoter for example an exogenous, constitutive or inducible promoter.

36. Kit according to Claim 35, **characterized in that** recombinant cells are recombinant fibroblasts, especially skin fibroblasts.

37. Kit according to Claim 35 **characterized in that** the recombinant cells are tumor cells.

38. Kit according to any one of the Claims 35 to 37, **characterized in that** the recombinant cells contain one exogenous nucleotide sequence coding for a protein whose expression is desired to be obtained, in particular, for a protein soluble in the serum.

39. Kit according to any one of Claims 35 to 37, **characterized in that** recombinant cells contain an exogenous nucleotide sequence coding for β-glucuronidase.

40. Kit according to Claim 31, **characterized in that** it comprises a recombinant retroviral vector comprising a proviral DNA sequence being modified in a manner such that:
- the gag, pol and env genes of the proviral DNA have been deleted at least in part in order to produce a proviral DNA incapable of replicating, this DNA being in addition incapable of recombining to form a wild-type virus,
- the LTR sequence contains a deletion in the sequence U3 such that transcription of the mRNA that it controls is reduced significantly for example at least 10-fold, and the recombinant retroviral vector comprises, in addition, an exogenous nucleotide sequence under the control of a promoter for example an exogenous, constitutive or inducible promoter.

41. Kit according to Claim 40, **characterized in that** the modified proviral DNA sequence, the exogenous nucleotide sequence and the exogenous promoter sequence are carried by a plasmid.

42. Kit according to Claim 40, **characterized in that** the proviral DNA is derived from the MuLV retrovirus.

43. Kit according to Claim 40 **characterized in that** the sequences of the pol and env genes of the proviral DNA are entirely deleted.

44. Kit according to any one of the Claims 40 to 43, **characterized in that** the U3 region of the LTR3' fragment in the retroviral vector is deleted at the level of nucleotide 2797 of Figure 1.

45. Kit according to any one of the Claims 40 to 43, **characterized in that** in the retroviral vector, the exogenous nucleotide sequence is under the control of the mouse PGK-1 promoter or the human PGK-1 promoter, optionally lacking a "TATA box".

46. Kit according to any one of the Claims 40 to 44, **characterized in that** in the retroviral vector, the proviral sequence upstream from the exogenous promoter is the proviral nucleotide sequence situated between nucleotides 1 and about 1500 of the sequence shown in figure 1.

47. Kit according to Claim 46, **characterized in that** in the retroviral vector, the exogenous nucleotide sequence is inserted at the BamHI site downstream the exogenous constitutive PGK-1 promoter.

48. Kit according to anyone of Claims 40 to 46, **characterized in that** the retroviral vector is the vector pM48 shown in Figure 2, modified by the insertion of the exogenous nucleotide sequence at the BamHI site.

49. Kit according to any one of Claims 39 to 45, **characterized in that** the retroviral vector contains at the BamHI site downstream the constitutive exogenous promoter, a BamHI fragment of the B-galactosidase gene.

50. Kit according to any one of Claims 40 to 49, **characterized in that** the retroviral vector further contains, upstream the constitutive exogenous promoter, an enhancer sequence.

## Patentansprüche

1. Implantat, **dadurch gekennzeichnet, dass** es umfasst:
- einen biokompatiblen Träger auf der Basis von Kalk, welcher die biologische Verankerung von Zellen erlaubt,
- Zellen, welche die Fähigkeit aufweisen, von Natur aus oder nach Rekombination eine bestimmte Verbindung, beispielsweise eine Verbindung, an welcher ein therapeutisches Interesse besteht, zu exprimieren und zu sezernieren; und
- einen Bestandteil, der in der Lage ist, die Gelbildung der genannten Zellen zu induzieren und/oder zu begünstigen.

2. Implantat nach Anspruch 1, **dadurch gekennzeichnet, dass** der biokompatible Träger ein Träger des. wenigstens teilweise, in vivo resorbierbaren Typs ist.

3. Implantat nach einem der Ansprüche 1 bis 2, **dadurch gekennzeichnet, dass** der biokompatible Träger ein Träger auf der Basis von Calciumcarbonat ist, wobei es sich vorzugsweise um Koralle handelt.

4. Implantat nach Anspruch 3 **dadurch gekennzeichnet, dass** der biokompatible Träger aus Koralle mit hoher Porosität besteht.

5. Implantat nach Anspruch 4, **dadurch gekennzeichnet, dass** die Koralle mit hoher Porosität aus einer kugelförmigen Koralle besteht.

6. Implantat nach einem der Ansprüche 1 bis 5, **dadurch gekennzeichnet, dass** der Bestandteil, welcher in der Lage ist, die Gelbildung der Zellen zu induzieren und/oder zu begünstigen, aus Kollagen, insbesondere aus Kollagen des Typs I, vorzugsweise in einer Konzentration in der Größenordnung von 1,5 mg/ml, besteht.

7. Implantat nach einem der Ansprüche 1 bis 6, **dadurch gekennzeichnet, dass** der Bestandteil, der in der Lage ist, die Gelbildung der Zellen zu induzieren und/oder zu begünstigen, ausgewählt ist aus Trägern auf der Basis von:
- nicht vernetztem Kollagen,
- Alginaten.

8. Implantat, **dadurch gekennzeichnet, dass** es umfasst:
- einen biokompatiblen Träger auf der Basis von Koralle, welcher die biologische Verankerung von Zellen erlaubt,
- rekombinante Zellen, welche die Fähigkeit aufweisen, eine bestimmte Verbindung zu exprimieren und zu sezernieren, und
- Kollagen vom Typ I.

9. Implantat nach einem der Ansprüche 1 bis 8, **dadurch gekennzeichnet, dass** die Zellen rekombinante Zellen, welche die Fähigkeit aufweisen, von einem Organismus, welchem sie verabreicht werden, immunologisch toleriert zu werden, sind, die durch eine Nukleotidsequenz modifiziert sind, welche für ein bestimmtes Polypeptid codiert.

10. Implantat nach Anspruch 9, **dadurch gekennzeichnet, dass** die rekombinanten Zellen Fibroblasten, insbesondere Hautfibroblasten, sind.

11. Implantat nach einem der Ansprüche 1 bis 10, **dadurch gekennzeichnet, dass** die genannten Zellen rekombinante Zellen sind, welche durch einen retroviralen Vektor modifiziert sind, der eine DNA-Sequenz eines Provirus umfasst, welche auf eine solche Weise modifiziert ist, dass:
- die Gene gag, pol und env der DNA des Provirus wenigstens teilweise supprimiert wurden und dass, um eine provirale DNA zu erhalten, welche nicht in der Lage ist, sich zu replizieren, diese DNA sich unter anderem nicht rekombinieren lässt, um einen Wildtyp-Virus zu bilden,
- die LTR-Sequenz eine Deletion in der Sequenz U3 trägt, so dass die Transkription in die mRNA-Form, welche diese steuert, in signifikanter Weise, beispielsweise wenigstens 10-fach, verringert ist und der retrovirale Vektor unter anderem eine exogene Nukleotidsequenz unter der Kontrolle eines Promotors, beispielsweise eines konstitutiven oder induzierbaren exogenen Promotors, umfasst.

12. Implantat nach Anspruch 11, **dadurch gekennzeichnet, dass** die provirale DNA des Vektors aus dem Retrovirus MuLV stammt.

13. Implantat nach Anspruch 10 oder Anspruch 11, **dadurch gekennzeichnet, dass** die Sequenzen der Gene pol und env der proviralen DNA vollständig supprimiert sind.

14. Implantat nach einem der Ansprüche 10 bis 12, **dadurch gekennzeichnet, dass** die Region U3 des Fragments LTR3' der proviralen DNA auf der Höhe des Nukleotids 2797 der Figur 1 supprimiert ist.

15. Implantat nach einem der Ansprüche 11 bis 14, **dadurch gekennzeichnet, dass** die exogene Nukleotidsequenz sich unter der Kontrolle des Mauspromotors PGK-1 oder des menschlichen Promotors PGK-1 befindet, gegebenenfalls ohne "TATA-Box".

16. Implantat nach einem der Ansprüche 11 bis 15, **dadurch gekennzeichnet, dass** die provirale Sequenz stromaufwärts des exogenen Promotors die Nukleotidsequenz des Provirus ist, die ungefähr zwischen den Nukleotiden 1 und 1500 der in Figur 1 dargestellten Sequenz liegt.

17. Implantat nach einem der Ansprüche 11 bis 16, **dadurch gekennzeichnet, dass** der retrovirale Vektor der Vektor pM48 ist, der in Figur 2 dargestellt ist, welcher durch die Einfügung der exogenen Nukleotidsequenz an der BamHI-Stelle modifiziert ist.

18. Implantat nach einem der Ansprüche 1 bis 9 oder 11 bis 17, **dadurch gekennzeichnet, dass** die rekombinanten Zellen Tumorzellen sind.

19. Implantat nach einem der Ansprüche 1 bis 18, **dadurch gekennzeichnet, dass** die rekombinanten Zellen durch einen Vektor modifiziert sind, welcher eine oder mehrere exogene Nukleotidsequenzen enthält, die für ein Antigen oder eine antigene Determinante codieren oder für ein Polypeptid oder ein Glycoprotein codieren, das in dem Serum löslich ist, beispielsweise für ein Polypeptid oder ein Glycoprotein von therapeutischem Interesse, insbesondere für ein Hormon, ein Strukturprotein oder ein -glycoprotein oder ein Protein oder ein Glycoprotein des Metabolismus oder ein virales Protein oder ein Glycoprotein oder ein Protein, welches die Eigenschaften eines Antikörpers oder eines Antikörperfragments aufweist.

20. Implantat nach einem der Ansprüche 1 bis 19, **dadurch gekennzeichnet, dass** es unter anderem einen oder mehrere angiogene Faktoren, insbesondere bFGF, enthält.

21. Implantat nach einem der Ansprüche 1 bis 20, **dadurch gekennzeichnet, dass** es Heparin oder ein Heparinderivat enthält.

22. Implantat nach einem der Ansprüche 1 bis 21, **dadurch gekennzeichnet, dass** es 10⁶ bis 10⁹, vorzugsweise 5 x 10⁶ bis 10⁷, rekombinante Zellen umfasst.

23. Verwendung eines Implantats nach einem der Ansprüche 1 bis 22 zur Herstellung einer Zusammensetzung, die zur permanenten oder vorübergehenden Implantation beim Menschen oder bei einem Tier bestimmt ist.

24. Zusammensetzung, **dadurch gekennzeichnet, dass** diese ein Implantat nach einem der Ansprüche 1 bis 22 zusammen mit einer oder mehreren Substanzen, insbesondere einem Antigen oder einem Hilfsmittel, umfasst.

25. Verwendung eines Implantats nach einem der Ansprüche 1 bis 22 zur Herstellung einer Verbindung, die bei der Behandlung genetischer Krankheiten, erworbener Krankheiten oder von Tumoren wirksam ist.

26. Verwendung eines Implantats nach einem der Ansprüche 1 bis 22 zur Herstellung einer Zusammensetzung, die zur Sekretion von Erythropoietin bestimmt ist.

27. Verfahren zur Herstellung eines Implantats nach einem der Ansprüche 1 bis 22, wobei das genannte Verfahren die folgenden Schritte umfasst:
- Inkontaktbringen des biokompatiblen Trägers auf der Basis von Kalk mit den genannten Zellen und einem Bestandteil, der in der Lage ist, deren Gelbildung zu induzieren und/oder zu begünstigen;
- Inkubation der im vorhergehenden Schritt erhaltenen Präparation, um die Gelbildung der genannten Bestandteile zu erhalten;
- Kultivierung der so erhaltenen Zellen unter Bedingungen, welche diesen erlauben, an den gelierten Bestandteilen anzuhaften, und
- Gewinnung des so erhaltenen Implantats.

28. Verfahren nach Anspruch 27, **dadurch gekennzeichnet, dass** der biokompatible Träger mit Zellen in Kontakt gebracht wird, welche vorher in eine Kollagenlösung eingearbeitet wurden.

29. Verfahren nach Anspruch 27 oder 28, **dadurch gekennzeichnet, dass** der biokompatible Träger aus Korallenpulver gebildet ist, das vorher mit einer Kollagenlösung und einem Wachstumsfaktor behandelt wurde.

30. Kit zur Herstellung eines Implantats, um die Expression und die Sekretion in vivo einer Verbindung durch Zellen zu realisieren, um eine gewünschte therapeutische Wirkung zu erzeugen, wobei das Kit umfasst:
- einen biokompatiblen Träger auf der Basis von Kalk, welcher die biologische Verankerung der genannten Zellen erlaubt, und
- einen Bestandteil, der in der Lage ist, die Gelbildung der genannten Zellen zu induzieren und/oder zu begünstigen.

31. Kit nach Anspruch 30, **dadurch gekennzeichnet, dass** der biokompatible Träger ein Träger auf der Basis von Caiciumcarbonat, vorzugsweise von Koralle, ist.

32. Kit nach Anspruch 30 oder 31, **dadurch gekennzeichnet, dass** der Bestandteil, der in der Lage ist, die Gelbildung der zellen zu induzieren und/oder zu begünstigen, aus Kollagen, insbesondere aus Kollagen des Typs I, vorzugsweise in einer Konzentration in der Größenordnung von 1,5 mg/ml, besteht.

33. Kit nach Anspruch 30, **dadurch gekennzeichnet, dass** dieses eine DNA umfasst, welche eine Sequenz umfasst, die für die von den genannten Zellen exprimierte und sezernierte Verbindung codiert.

34. Kit nach einem der Ansprüche 30 bis 33, **dadurch gekennzeichnet, dass** dieses Zellen umfasst, welche von Natur aus oder nach Rekombination die Fähigkeit aufweisen, eine bestimmte Verbindung, beispielsweise eine Verbindung, an welcher ein therapeutisches Interesse besteht, zu exprimieren und zu sezernieren.

35. Kit nach Anspruch 34, **dadurch gekennzeichnet, dass** die Zellen rekombinante Zellen, welche die Fähigkeit aufweisen, von einem Organismus, welchem sie verabreicht werden, immunologisch toleriert zu werden, sind, die durch einen retroviralen Vektor modifiziert sind, welcher umfasst:
eine DNA-Sequenz eines Provirus, welche auf eine solche Weise modifiziert ist, dass:
- die Gene gag, pol und env der DNA des Provirus wenigstens teilweise deletiert wurden und dass, um eine provirale DNA zu erhalten, welche nicht in der Lage ist, sich zu replizieren, diese DNA sich unter anderem nicht rekombinieren lässt, um einen wildtyp-Virus zu bilden,
- die LTR-Sequenz eine Deletion in der Sequenz U3 trägt, so dass die Transkription in die mRNA-Form, welche diese steuert, in signifikanter Weise, beispielsweise wenigstens 10-fach, verringert ist, und
der rekombinante retrovirale Vektor unter anderem eine exogene Nukleotidsequenz unter der Kontrolle eines Promotors, beispielsweise eines konstitutiven oder induzierbaren exogenen Promotors, umfasst.

36. Kit nach Anspruch 35, **dadurch gekennzeichnet, dass** die rekombinanten Zellen rekombinante Fibroblasten, insbesondere Hautfibroblasten, sind.

37. Kit nach Anspruch 35, **dadurch gekennzeichnet, dass** die rekombinanten Zellen Tumorzellen sind.

38. Kit nach einem der Ansprüche 35 bis 37, **dadurch gekennzeichnet, dass** die rekombinanten Zellen eine exogene Nukleotidsequenz enthalten, die für ein Protein, welches man exprimieren möchte, insbesondere für ein Protein, das im Serum löslich ist, codiert.

39. Kit nach einem der Ansprüche 35 bis 37, **dadurch gekennzeichnet, dass** die rekombinanten Zellen eine exogene Nukleotidsequenz enthalten, die für die β-Glucuronidase codiert.

40. Kit nach Anspruch 31, **dadurch gekennzeichnet, dass** dieses einen rekombinanten retroviralen Vektor umfasst, welcher umfasst:
eine DNA-Sequenz eines Provirus, welche in einer solchen Weise modifiziert ist, dass:
- die Gene gag, pol und env der DNA des Provirus wenigstens teilweise deletiert wurden und dass, um eine provirale DNA zu erhalten, welche nicht in der Lage ist, sich zu replizieren, diese DNA sich unter anderem nicht rekombinieren lässt, um einen Wildtyp-Virus zu bilden,
- die LTR-Sequenz eine Deletion in der Sequenz U3 trägt, so dass die Transkription in die mRNA-Form, welche diese steuert, in signifikanter Weise, beispielsweise wenigstens 10-fach, verringert ist, und
der rekombinante retrovirale Vektor unter anderem eine exogene Nukleotidsequenz unter der Kontrolle eines Promotors, beispielsweise eines konstitutiven oder induzierbaren exogenen Promotors, umfasst.

41. Kit nach Anspruch 40, **dadurch gekennzeichnet, dass** die modifizierte DNA-Sequenz des Provirus, die exogene Nukleotidsequenz und der exogene Promotor von einem Plasmid getragen werden.

42. Kit nach Anspruch 40, **dadurch gekennzeichnet, dass** die provirale DNA von dem Retrovirus MuLV stammt.

43. Kit nach Anspruch 40, **dadurch gekennzeichnet, dass** die Sequenzen der Gene pol und env der proviralen DNA vollständig supprimiert sind.

44. Kit nach einem der Ansprüche 40 bis 43, **dadurch gekennzeichnet, dass** in dem retroviralen Vektor die Region U3 des Fragments LTR3' auf der Höhe des Nukleotids 2797 von Figur 1 supprimiert ist.

45. Kit nach einem der Ansprüche 40 bis 43, **dadurch gekennzeichnet, dass** in dem retroviralen Vektor die exogene Nukleotidsequenz sich unter der Kontrolle des Mauspromotors PGK-1 oder des menschlichen Promotors PGK-1 befindet, gegebenenfalls ohne "TATA-Box".

46. Kit nach einem der Ansprüche 40 bis 44, **dadurch gekennzeichnet, dass** in dem retroviralen Vektor die provirale Sequenz stromaufwärts des exogenen Promotors die Nukleotidsequenz des Provirus ist, die ungefähr zwischen den Nukleotiden 1 und 1500 der in Figur 1 dargestellten Sequenz liegt.

47. Kit nach Anspruch 46, **dadurch gekennzeichnet, dass** in dem retroviralen Vektor die exogene Nukleotidsequenz an der BamHI-Stelle stromabwärts des exogenen konstitutiven Promotors PGK-1 eingefügt ist.

48. Kit nach einem der Ansprüche 40 bis 46, **dadurch gekennzeichnet, dass** der retrovirale Vektor der Vektor pM48 ist, welcher in Figur 2 dargestellt ist, welcher durch die Einfügung der exogenen Nukleotidsequenz an der BamHI-Stelle modifiziert ist.

49. Kit nach einem der Ansprüche 39 bis 45, **dadurch gekennzeichnet, dass** der retrovirale Vektor an der BamHI-Stelle stromabwärts des exogenen konstitutiven Promotors ein Bam-HI-Fragment des Gens der β-Galactosidase enthält.

50. Kit nach einem der Ansprüche 40 bis 49, **dadurch gekennzeichnet, dass** der retrovirale Vektor unter anderem stromaufwärts des exogene konstitutiven Promotors eine Aktivatorseguenz enthält.
